(19) Europäisches Patentamt
European Patent Office
Office européen des brevets

(11) **EP 2 834 278 B1**

(12) **EUROPEAN PATENT SPECIFICATION**

(45) Date of publication and mention of the grant of the patent:
**15.03.2017 Bulletin 2017/11**

(51) Int Cl.:
*C08F 122/02* (2006.01)     *A62D 3/30* (2007.01)
*C08F 8/10* (2006.01)       *C08F 290/04* (2006.01)
*C08F 220/06* (2006.01)     *C08F 226/06* (2006.01)
*C08F 222/10* (2006.01)

(21) Application number: **13715734.3**

(22) Date of filing: **05.04.2013**

(86) International application number:
**PCT/GB2013/050896**

(87) International publication number:
**WO 2013/150317 (10.10.2013 Gazette 2013/41)**

(54) **DECONTAMINANT PRODUCT AND METHOD**

DEKONTAMINATIONSPRODUKT UND VERFAHREN

PRODUIT DÉCONTAMINANT ET PROCÉDÉ ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.04.2012 GB 201206190**

(43) Date of publication of application:
**11.02.2015 Bulletin 2015/07**

(73) Proprietor: **The Secretary of State for Health London SW1A 2NS (GB)**

(72) Inventor: **CHILCOTT, Robert Hatfield Hertfordshire AL10 9AB (GB)**

(74) Representative: **Griffin, Philippa Jane et al Mathys & Squire LLP The Shard 32 London Bridge Street London SE1 9SG (GB)**

(56) References cited:
EP-A1- 0 968 765     WO-A1-2008/125887
US-A- 5 100 477     US-A1- 2010 036 188

• REDDITHOTA J KRUPADAM ET AL: "Removal of cyanotoxins from surface water resources using reusable molecularly imprinted polymer adsorbents", ENVIRONMENTAL SCIENCE AND POLLUTION RESEARCH - INTERNATIONAL, SPRINGER BERLIN / HEIDELBERG, LA, vol. 19, no. 5, 30 December 2011 (2011-12-30), pages 1841-1851, XP035078546, ISSN: 1614-7499, DOI: 10.1007/S11356-011-0703-1

• MAHATO T H ET AL: "Nanocrystalline zinc oxide for the decontamination of sarin", JOURNAL OF HAZARDOUS MATERIALS, ELSEVIER, AMSTERDAM, NL, vol. 165, no. 1-3, 15 June 2009 (2009-06-15), pages 928-932, XP026053795, ISSN: 0304-3894, DOI: 10.1016/J.JHAZMAT.2008.10.126 [retrieved on 2008-11-11]

• SERGEYEVA T A ET AL: "Catalytic molecularly imprinted polymer membranes: Development of the biomimetic sensor for phenols detection", ANALYTICA CHIMICA ACTA, ELSEVIER, AMSTERDAM, NL, vol. 659, no. 1-2, 5 February 2010 (2010-02-05), pages 274-279, XP026819681, ISSN: 0003-2670 [retrieved on 2009-12-04]

**Description**

The present invention relates to a decontaminant product, decontamination kit, and related methods and uses for decontamination or for protection against contamination, as defined in the claims.

Decontamination following exposure to potentially hazardous or toxic chemical agents, such as chemical warfare agents, is an important task performed by military personnel, first responders and ambulance staff at the scene of an incident. It requires rapid action and immediate treatment to ensure effective decontamination of hazardous agents, to reduce the risk of harm to exposed individuals, and to reduce the risk of damage to the environment, clothing and important equipment (eg. military or laboratory equipment).

In addition to performing decontamination following an exposure incident, it is also important to protect individuals at high risk of exposure to hazardous agents; this includes individuals working with chemical agents in laboratories or factories, and individuals (eg. military personal) at risk of exposure to hazardous chemical agents (eg. warfare agents). At present, individuals working with hazardous agents are provided with protective equipment (eg. gloves, safety glasses, laboratory coats); however further development of protective equipment that actively decontaminates chemical agents would help to reduce the risk of harm in the event of a chemical exposure incident.

A number of products are commercially available for decontamination of hazardous chemical agents, including Fuller's Earth (used currently by the UK military) and Reactive Skin Decontamination Lotion (RSDL) (used for military applications worldwide).

Fuller's Earth is a natural form of aluminium silicate (clay). The efficacy of Fuller's Earth as a chemical decontaminant is related to its processing into a fine powder, which increases its surface area and subsequently its passive absorbance capability. The powder is spread onto contaminated surfaces to absorb chemical agents, and then collected (typically swept up) and disposed of via incineration. The use of Fuller's Earth powder is associated with a number of drawbacks. It can be difficult to apply Fuller's Earth powder to an exposed site/ surface without the use of an applicator, and the powder may be difficult to contain/ confine at the exposed site/ surface (eg. the powder may fall off or be blown off), therefore limiting its efficacy. Additionally, the scattered/ dispersed powder may pose an inhalation hazard and an irritant for the eyes and lungs of exposed individuals. Fuller's Earth must be mined and stockpiled, and thus the resources of this product are limited.

Reactive Skin Decontamination Lotion (RSDL) is an oxime-based topical decontamination formulation containing the active ingredient 2,3 butadiene monoxime (DAM), dissolved in a solvent. The RSDL formulation actively desorbs, retains and sequesters contaminating chemical agents, which are then neutralised by the active ingredient via a nucleophilic reaction.

RSDL has been demonstrated to be effective against a range of common hazardous chemical agents. However, application of RSDL directly to skin (in the presence of sulphuric acid) has been observed to pose a burn hazard to skin. Thus, it is recommended that the RSDL lotion is used as part of a 2-step decontamination kit, which contains a dry wipe to remove excess hazardous chemical agents before applying the RSDL lotion via a sponge. In this regard, Step 1 involves physical removal of contaminants using the dry wipe, which permits adsorption and absorption of chemical agents from the contaminated surface (eg. skin). The dry wipe is made from porous and absorbent fabrics layered around a non-particulate fabric form of activated carbon. Step 2 involves scrubbing the wiped surface with the RSDL-saturated sponge, allowing the RSDL lotion to leave the sponge and neutralise any residual chemical agent left on the surface following wiping.

Although RSDL is effective against a range of chemical agents, the liquid formulation of this lotion restricts its suitability for use on water-sensitive equipment, and the lotion leaves slippery residue on the decontaminated surface (which can complicate the clean-up process and cause additional health and safety concerns). In addition, the shelf-life of an RSDL-saturated sponge may be limited.

WO 2008/125887 describes polymers capable of binding ochratoxins. The polymers may be used for solid phase extraction of ochratoxins and immobilisation of ochratoxins in solid phase extraction cartridges, for qualitative or quantitative analysis of ochratoxins in liquid extracts from foodstuffs or animal feeds.

US 5100477 describes a process of decontaminating a surface contaminated with a toxic chemical agent in which there is applied to the contaminated surface, a macroporous cross-linked hydrophobic co-polymer containing an agent which is a decontaminant for the toxic chemical agent present on the surface.

Reddithota et al. (Environmental Science and Pollution Research - International, Springer Berlin/Heidelberg, Vol 19, no. 5, 30 December 2011, pages 1841-1851) describes the removal of cyanotoxins from surface water resources using reusable molecularly imprinted polymer absorbents.

There is therefore a need in the art for an improved decontaminant that avoids one or more of the technical problems associated with existing decontaminant formulations/ products. Preferably, the decontaminant has at least one of the recognised desirable characteristics for a decontaminant, including: suitability for a wide range of different agents; suitability for use on a range of surfaces (including delicate surfaces such as skin); safety (ie. non-toxic, non-irritant and/ or non-allergenic); rapid action; ease of use and disposal; long-term stability; and affordability.

The present invention solves this technical problem by providing a decontaminant product (as defined in the claims) for decontaminating a surface or object that is contaminated, or suspected to be contaminated, with a hazardous chemical agent; wherein the decontaminant product comprises polymeric material comprising a polymer of:

(i) itaconic acid or a derivative thereof, such as an itaconic acid ester or an itaconic acid isomer;
(ii) an acrylate monomer;
(iii) urocanic acid or a derivative thereof, such as an ester thereof;
(iv) a vinyl monomer; or
(v) an amine monomer;

wherein the decontaminant product is a sponge, gel, liquid, paste, ointment, lotion, cream, foam, or fabric product suitable for personal use.

As used herein, the term 'contaminant' means a potentially hazardous agent (as defined herein) that, if present on or in a surface or object, causes or is capable of causing temporary or permanent harm or damage to the surface or object (or to another surface, object or individual coming into contact with the contaminated surface or object) unless or until it at least partially detoxified, neutralised or removed from the surface or object. The term 'contamination' refers to the presence of a contaminant (as defined herein) on or in a surface or object. Contamination may occur due to accidental or deliberate release of or exposure to a contaminant.

As used herein, the terms "decontaminate" and "decontamination" relate to a process by which a contaminant (as defined herein) is at least partially detoxified, neutralised or destroyed, or at least partially removed or reduced in quantity, thereby reducing or eliminating potential harm or damage that may be caused by exposure to the agent. For example, the chemical structure of a contaminating hazardous agent may be modified by decontamination to a chemical structure that is less harmful or damaging. In one embodiment, the terms "decontaminate" and "decontamination" relate to the removal, or at least partial removal, of a contaminant such as a hazardous chemical agent from a surface or object.

As used herein, the term 'decontaminant product' means a composition, formulation, agent or item suitable for and capable of at least partially removing, detoxifying, neutralising or destroying a potentially hazardous agent, resulting in a reduction or elimination of potential harm or damage caused by exposure to the agent. In one embodiment, the 'decontaminant product' comprises or consists of a composition, formulation, agent or item suitable for and capable of removing, or at least partially removing, a contaminant such as a hazardous chemical agent from a surface or object. In one embodiment, said decontaminant product comprises or consists of an adsorbent composition, formulation, agent or item that adsorbs a contaminant such as a hazardous chemical agent from a surface or object, and thereby removes, or at least partially removes, said contaminant from the surface or object. The terms 'decontaminant product', 'decontamination product' and 'decontaminant' are synonymous and used interchangeably throughout.

The decontaminant product of the invention comprises (or consists of) polymeric material, as defined in the claims. Polymeric material comprises (or consists of) one or more polymers (as defined in the claims). The decontaminant product of the invention can thus be described as being 'polymeric', or as having a polymeric structure. In one embodiment, the 'polymeric' material is capable of adsorbing, or at least partially adsorbing a contaminant such as a hazardous chemical agent, and thus the decontaminant product of the invention can also be described as comprising (or consisting of) 'adsorbent material'. In accordance with this embodiment of the invention, the decontaminant product may be termed a 'polymeric adsorbent'.

A polymer is a molecule formed from repeating structural units (monomers) connected by covalent chemical bonds. Polymers are formed from monomers in polymerisation reactions. As used herein, the term monomer relates to a single structural unit used to generate a polymer.

Derivatives of itaconic acid include itaconic acid esters and isomers of itaconic acid. Examples of itaconic acid esters include alkyl esters (eg. alkylitaconate). Examples of itaconic acid isomers include mesaconic acid and citroconic acid.

Thus, in one embodiment, the decontaminant product comprises polymeric material comprising a polymer of itaconic acid, or a derivative thereof. In one embodiment, the polymeric material comprises a polymer of an itaconic acid ester or a polymer of an itaconic acid isomer. In one embodiment, the decontaminant product comprises polymeric material comprising a polymer of itaconic acid.

Acrylate monomers include monomer compounds such as 2-trifluoromethyl acrylic acid (TFMAA), methacrylic acid (MA) *N,N*-methylene bisacrylamide (MBA), 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, and acrolein.

Thus, in one embodiment, the decontaminant product comprises polymeric material comprising a polymer of an acrylate compound selected from the group consisting of 2-trifluoromethyl acrylic acid (TFMAA), methacrylic acid (MA), *N,N*-methylene bisacrylamid (MBA), 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, and acrolein.

In one embodiment, the decontaminant product comprises polymeric material comprising a polymer of an acrylate compound selected from the group consisting of 2-trifluoromethyl acrylic acid (TFMAA), methacrylic acid (MA) and *N,N*-methylene bisacrylamide (MBA). For example, the decontaminant product or polymeric adsorbent may comprise polymeric material comprising a polymer of 2-trifluoromethyl acrylic acid (TFMAA).

Derivatives of urocanic acid include urocanic acid esters, such as urocanic acid ethyl ester. Thus, in one embodiment, the decontaminant product comprises polymeric material comprising a polymer of urocanic acid, or a derivative thereof. In one embodiment, the polymeric material comprises a polymer of a urocanic acid ester, such as a polymer of urocanic acid ethyl ester. In one embodiment, the polymeric material comprises a polymer of urocanic acid.

Vinyl monomers include compounds such as 1-Vinylimidazole, $_p$-divinylbenzene, $_m$-Divinylbenzene, 2-Vinylpyridine and 4-Vinylpyridine. Thus, in one embodiment, the decontaminant product comprises polymeric material comprising a polymer of a vinyl monomer selected from the group consisting of 1-Vinylimidazole, $_p$-divinylbenzene, $_m$-Divinylbenzene, 2-Vinylpyridine and 4-Vinylpyridine.

Amine monomers include compounds such as allylamine. Thus, in one embodiment, the decontaminant product comprises polymeric material comprising a polymer of an amine compound, such as a polymer of allylamine.

In one embodiment, the decontaminant product (as defined in the claims) comprises polymeric material comprising a polymer of:

(i) itaconic acid, or a derivative thereof such as an itaconic ester or an itaconic acid isomer, such as mesaconic acid or citraconic acid;

(ii) an acrylate monomer, such as 2-trifluoromethyl acrylic acid (TFMAA), methacrylic acid, *N,N*-methylene bisacrylamide, 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, acrolein, or a derivative thereof;

(iii) urocanic acid, or a derivative thereof such as an ester thereof, such as urocanic acid ethyl ester;

(iv) a vinyl monomer, such as 1-Vinylimidazole, $_p$-divinylbenzene, $_m$-Divinylbenzene, 2-Vinylpyridine, 4-Vinylpyridine; or

(v) an amine monomer, such as allylamine.

In one embodiment, the decontaminant product (as defined in the claims) comprises polymeric material comprising a polymer of:

(i) itaconic acid, or a derivative thereof such as an itaconic ester or an itaconic acid isomer, such as mesaconic acid or citraconic acid;

(ii) an acrylate monomer selected from the group consisting of 2-trifluoromethyl acrylic acid (TFMAA), methacrylic acid, *N,N*-methylene bisacrylamide, 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, acrolein, or a derivative thereof;

(iii) urocanic acid, or a derivative thereof such as an ester thereof, such as urocanic acid ethyl ester;

(iv) a vinyl monomer selected from the group consisting of 1-Vinylimidazole, $_p$-divinylbenzene, $_m$-Divinylbenzene, 2-Vinylpyridine, 4-Vinylpyridine; or

(v) allylamine.

In one embodiment, the decontaminant product (as defined in the claims) comprises polymeric material comprising a polymer of:

(i) itaconic acid, or a derivative thereof such as an itaconic ester or an itaconic acid isomer, such as mesaconic acid or citraconic acid;

(ii) an acrylate monomer selected from the group consisting of 2-trifluoromethyl acrylic acid (TFMAA), methacrylic acid (MA) and *N,N*-methylene bisacrylamide (MBA); or

(iii) urocanic acid.

In one embodiment, the polymeric material comprises a polymer of itaconic acid (IA), 2-trifluoromethyl acrylic acid (TFMAA), methacrylic acid (MA), *N,N*-methylene bisacrylamide (MBA) or urocanic acid. Thus, in one embodiment, the polymer is a itaconic acid polymer, a TFMAA polymer, a methacrylic acid polymer, an *N,N*-methylene bisacrylamide polymer, or a urocanic acid polymer.

In one embodiment, the polymer is a polymer of itaconic acid or a polymer of 2-trifluoromethyl acrylic acid. For example, the polymer may be a polymer of itaconic acid. Alternatively, the polymer may be a polymer of 2-trifluoromethyl acrylic acid.

The monomer units used to form the polymer may be the same or different.

Thus, in one embodiment, the polymer is a homopolymer (ie. the polymer is formed from repeating units of the same monomer). By way of example, the polymer may be formed from repeating units of itaconic acid (ie. an itaconic acid homopolymer), or from repeating units of 2-trifluoromethyl acrylic acid (ie. a TFMAA homopolymer).

In an alternative embodiment, the polymer is a co-polymer (ie. the polymer is formed from at least two different monomers, such as at least 2, 3, 4 or 5 different monomers). By way of example, the polymer may be formed from repeating units of itaconic acid and a different monomer (ie. an itaconic acid co-polymer); or from repeating units of 2-trifluoromethyl acrylic acid and a different monomer (ie. a TFMAA co-polymer).

Thus, as used herein, the term "polymer" embraces both homopolymers and co-polymers. For example, the term "itaconic acid polymer" (or "polymer of itaconic acid") embraces both itaconic acid homopolymers and itaconic acid co-polymers.

In one embodiment, the polymer is a cross-linked polymer. Cross-linked polymers may be homopolymers or co-polymers. In an alternative embodiment, the polymer is a linear polymer. Linear polymers may be homopolymers or co-polymers.

In one embodiment, the polymeric material comprises (or consists of) only one type of polymer. For example, all the polymer molecules in the polymeric material are formed from the same monomer(s). In accordance with this embodiment, the polymeric material is homogeneous with respect to its polymer content.

In an alternative embodiment, the polymeric material comprises (or consists of) multiple different polymers (such as at least 2, 3, 4 or 5 different polymers). For example, the different polymers molecules in the polymeric material may be formed from different monomers. In accordance with this embodiment, the polymeric material is heterogeneous with respect to its polymer content. In one embodiment, the polymeric material may comprise a mixture of homopolymers and co-polymers. In one embodiment, the polymeric material may comprise a mixture of cross-linked polymers and linear polymers, which may each be either homopolymers or co-polymers.

In one embodiment, the polymeric material comprises one polymer (eg. an IA polymer) attached to (eg. bound to, grafted to or cross-linked to) one or more different polymers. For example, the polymeric material may comprise a polymer grafted onto a 'backbone' comprising one or more different polymers (eg. polyethylene glycol, PEG). In one embodiment, the polymeric material comprises an IA polymer grafted onto PEG. The inventors have identified that polymeric material comprising a polymer (eg. a polymer of IA) grafted onto a PEG backbone is particularly suitable in the 'sponge' and 'powder' aspects of the decontaminant product (discussed below). In this regard, the inclusion of additional polymers into the polymeric material (eg. polyethylene glycol, PEG) may be important for facilitating the correct molecular spacing between docking groups within the polymeric material, which may improve the efficacy of the decontaminant product for binding and sequestering (eg. adsorbing) chemical contaminant agents.

In one embodiment, the decontaminant product of the invention (having any of the forms defined in the claims) further comprises a transition metal such as zirconium, titanium, manganese, magnesium, calcium, or aluminium, (or a salt thereof, eg. an oxide), which may be incorporated into the polymeric material. For example, the decontaminant product may comprise zirconium in the form of a zirconium salt, such as zirconium oxide. In this regard, the inventors have found that the addition of zirconium (eg. optionally in the form of a salt such as zirconium oxide) provides additional chelating capacity, which may increase the decontamination efficacy, particularly for decontamination of organophosphate-based contaminants such as GD and VX.

Fuller's Earth comprises transition metals and thus the decontamination product of the invention (as defined in the claims) may in one embodiment further comprise Fuller's Earth, combined with the polymeric material.

Unlike Fuller's Earth, which exerts its decontaminating effect via a passive mechanism (absorption), the polymeric decontaminant product of the present invention (as defined in the claims) uses an active mechanism for decontamination. In this regard, the decontaminant product of the invention comprises a polymer that binds and sequesters potentially hazardous chemical agents such as CWAs via interactions such as ionic bonds, hydrogen bonds, van der Waals' forces, dipole-dipole interactions, and/ or steric factors. In one embodiment, the polymer binds the contaminating agent with high affinity (eg. with an affinity in the region of about -4 to about -55 Kcal mol$^{-1}$). In one embodiment, the decontaminant product of the invention acts by adsorption. In accordance with this embodiment, the decontaminant product comprises polymeric material (as defined in the claims) that adsorbs the contaminant (eg. hazardous chemical agent) from the contaminated surface or object. In accordance with this embodiment of the invention, the decontaminant product may be termed a 'polymeric adsorbent'. In one embodiment, the polymeric adsorbent decontaminant product binds and sequesters the contaminant with high affinity.

In one embodiment, the polymer molecules present in the decontaminant product of the invention are too large to traverse the skin barrier, and therefore have a low risk of skin irritation (as confirmed in independent skin toxicology reports conducted by Harlan laboratories). For example, the polymer molecules may be at least 250, 300, 400 or 500Da.

The decontaminant product of the invention has many applications and can be used in many different environments, and is particularly suited for use where exposure to hazardous chemical agents (eg. chemical warfare agents, "CWAs") is a concern.

The decontaminant product of the present invention is suitable for decontamination following release of/ exposure to a hazardous chemical agent (eg. chemical warfare agent, CWA). Thus, another term for the decontaminant product of the invention is a "hazardous agent decontaminant", or more specifically a "hazardous chemical decontaminant", or a "CWA decontaminant"

The decontaminant product of the present invention (as defined in the claims) is suitable for decontamination of a surface or object that is contaminated, or suspected to be contaminated, with a hazardous chemical agent. In accordance with this embodiment of the invention, the decontaminant product may be termed a "hazardous chemical decontaminant" or a "hazardous chemical decontamination product". In one embodiment, the decontamination product comprises or consists of polymeric material (as defined in the claims) that is capable of adsorbing a hazardous chemical agent from a surface or object. This polymeric adsorbent of the invention is thus suitable for decontaminating a surface or object contaminated (or suspected to be contaminated) with said hazardous chemical agent.

As used herein, the term hazardous chemical agent means any chemical compound, constituent, species or agent that causes or is capable of causing temporary or permanent harm (eg. injury, illness or death) to humans or animals, or that causes or is capable of causing damage to the environment (eg. to plants, water or soil), or to equipment (eg. corrosion of metal).

Examples of hazardous chemical agents that can be decontaminated in accordance with the present invention include chemical warfare agents (CWAs), and chemical surrogates thereof. Thus, in one embodiment, the decontaminant product of the invention is a 'CWA decontaminant'/ 'CWA decontamination product'. In one embodiment, the polymeric adsorbent of the invention comprises or consists of polymeric material that adsorbs a chemical warfare agent.

Examples of chemical warfare agents include blister agents and nerve agents. The term blister agent includes urticants, and vesicants such as sulphur mustards, nitrogen mustards and arsenicals. Examples of Sulphur Mustards include bis(2-chloroethyl(sulphide)) (HD), 2-chloroethylchloromethylsulphide, bis(2-chloroethylthio)methane, 1,2-bis(2-chloroethylthio)ethane, 1,3-bis(2-chloroethylthio)-n-propane, 1,4-bis(2-chloroethylthio)-n-butane, 1,5-bis(2-chloeoethylthio)-n-pentane, bis(2-chloroethylthiomethyl)ether, and bis(2-chloroethylthioethyl)ether)).

Methyl salicylate (MeS) is used as a stimulant/ surrogate for the research of the chemical warfare agent Sulphur Mustard, due to its similar chemical and physical properties.

The term nerve agent includes G series, GV series and V series agents. Examples of G series nerve agents include o-alkyl phosphofluoridates (such as propan-2-yl methylphosphonofluoridate (Sarin, GB), cyclosarin (GF), and 3,3-dimethylbutan-2-yl methylphosphonofluoridate (Soman, GD)); and o-alkyl phosphoramindcyanidates (such as Tabun (GA)). Examples of V series nerve agents include o-alkyl, s-2-dialkyl aminoethyl alkylphosphothiolates and corresponding salts such as VX (Ethyl ({2-[bis(propan-2-yl)amino]ethyl}sulfanyl)(methyl)phosphinate) and related agents VG, VE and VM.

In one embodiment, decontaminant product of the present invention is suitable for decontamination of Methyl salicylate (MeS), sulphur mustard (HD, bis(2-chloroethyl) sulphide), VX (O-ethyl-S-(2-(diisopropylamino)ethyl)methylphophonothioate), Soman (GD, pinacolyl methylphosphonofluoridate), Sarin (GB, isopropyl methylphosphonofluoridate), or Tabun (GA, ethyl *N,N* dimethylphosphoramidocyanidate.

Other examples of hazardous chemical agents that can be decontaminated in accordance with the present invention include toxic industrial chemicals, domestic chemicals, organophosphorus compounds, and pesticides (eg. insecticides and herbicides).

In one embodiment, the decontaminant product of the invention is capable of removing, detoxifying, or neutralising at least 5% (eg. at least 10, 15, 20, 25, 30, 35, 40, 45, 50, 55, 60, 65, 70, 75, 80, 85, 90, 95 or 100%) of the potentially hazardous chemical agent. For example, the decontaminant product of the invention may be capable of removing, detoxifying, or neutralising at least 60% (eg. at least 65, 70, 75, 80, 85, 90, 95 or 100%) of the potentially hazardous chemical agent.

In one embodiment, the decontaminant product of the invention is capable of adsorbing at least 5% (eg. at least 10, 15, 20, 25, 30, 35, 40, 45, 50, 55, 60, 65, 70, 75, 80, 85, 90, 95 or 100%) of the potentially hazardous chemical agent. For example, the decontaminant product of the invention may be capable of adsorbing at least 60% (eg. at least 65, 70, 75, 80, 85, 90, 95 or 100%) of the potentially hazardous chemical agent.

In one embodiment, the decontaminant product of the invention is capable of reducing the potential harm or damage caused by exposure to the agent by at least 5% (eg. by at least 10, 15, 20, 25, 30, 35, 40, 45, 50, 55, 60, 65, 70, 75, 80, 85, 90, 95 or 100%). In one embodiment, the contamination remaining following use of the decontaminant product of the invention is less than 95% (eg. less than 90, 85, 80, 75, 70, 65, 60, 55, 50, 45, 40, 35, 30, 25, 20, 15, 10 or 5%) of the contamination present prior to use of the decontaminant product.

In addition, the decontaminant product of the invention may also have utility for other hazardous agents, such as biological agents.

The decontaminant product of the invention can be used to decontaminate a wide range of different surfaces, substrates or objects (eg. surfaces of substrates/ objects). The decontamination product of the invention can be used to decontaminate surfaces, substrates or objects composed of any material, including skin, hair, metal, plastic, fabric, fibre, glass, or ceramic, or any mixture of these.

Examples of surfaces or objects that can be decontaminated using the decontaminant product of the invention include (without limitation) the human or animal body, or a part thereof such as skin, hair or teeth; items of clothing; military equipment (such as armour, weapons or ammunition); laboratory equipment (such as laboratory benches, centrifuges, sinks); medical equipment (such as medical instruments and apparatus, and also hospital furnishings and floors); vehicles (such as aeroplanes, trains, cars or buses); building materials; and any other part of a public area (including buildings such as airports, stations, schools, arenas).

The decontaminant product of the invention is suitable for personal use, such as for decontamination of human skin, hair or clothing, or for decontamination of personal items/ equipment. In embodiments of the invention, the decontaminant product is small and/or portable (eg. of a size that fits in the human hand and/or can easily be carried by hand, in a pocket, or in a back-pack/ rucksack), and may thus advantageously be suited to field use.

The decontaminant product of the invention can take a number of different forms (as defined in the claims).

The present disclosure also provides a decontaminant product or polymeric adsorbent that comprises or consists of (or is in the form of) a powder or granules (ie. a powder or granular formulation). In accordance with this embodiment, the disclosure provides a decontaminant powder or a granular decontaminant product, such as a polymeric adsorbent powder, or a granular polymeric adsorbent. The decontaminant powder or granules comprise the polymeric material described herein.

The decontaminant product of the invention does not comprise or consist of powder or granules. The decontaminant product of the invention is not a powder or granular formulation.

The decontaminant product of the invention (as defined in the claims) comprises or consists of (or may be in the form of or be formulated as) a liquid, gel, paste, ointment, lotion, cream, or foam suitable for personal use. In one embodiment, the decontaminant product comprises or consists (or is in the form of or be formulated as) of an adsorbent liquid, gel, paste, ointment, lotion, cream or foam. In accordance with these embodiments, the composition of the liquid, gel, paste, ointment, lotion, cream, or foam may be chosen so that it is suitable for spreading without flowing from the hands. In one embodiment, the liquid, gel, paste, ointment, lotion, cream, or foam may be particularly suitable for decontamination of human skin, hair or clothing, or for decontamination of personal items/ equipment.

In one embodiment, the decontaminant product of the invention comprises or consists of (or is in the form of or is formulated as) a gel that is suitable for personal use. In accordance with this embodiment, the invention provides a decontaminant gel, such as a polymeric adsorbent gel. The decontaminant gel comprises the polymeric material of the invention (eg. dispersed within the gel). As used herein, the term 'gel' means a viscous liquid formulation. As used herein, the term "gel" is not limited to any particular viscosity. In one embodiment, the composition of the gel may be chosen so that it is given a suitable viscosity for good spreading in application without flowing from the hands. Gel formulations of the invention are particularly useful for decontamination of skin or hair.

In one embodiment, the decontaminant product in the form of a gel suitable for personal use comprises polymer particles in the 20-45$\mu$m size range dispersed within the gel. In one embodiment, the polymer particles in the 20-45$\mu$m size range are dispersed in PEG at a concentration of 0.1g-0.5g polymer/ ml PEG, such as from 0.1, 0.15, 0.2, 0.25, 0.3, 0.4 or 0.5g polymer/ ml PEG, such as at a concentration of about 0.23g polymer/ml PEG.

In one embodiment, the decontaminant product in the form of a liquid, gel, paste, ointment, lotion, cream, or foam suitable for personal use further comprises a transition metal such as zirconium, titanium, manganese, magnesium, calcium, or aluminium (or a salt thereof, eg. an oxide). For example, the liquid, gel, paste, ointment, lotion, cream, or foam may further comprise zirconium, or a zirconium salt, such as zirconium oxide.

The invention thus provides a decontaminant product of the invention in the form of a gel suitable for personal use, wherein said decontaminant product or polymeric adsorbent further comprises a transition metal such as zirconium, titanium, manganese, magnesium, calcium, or aluminium (or a salt thereof, eg. an oxide). For example, the gel may further comprise zirconium, or a zirconium salt, such as zirconium oxide.

In one embodiment, the decontaminant product of the invention, as defined in the claims, comprises or consists of (or is in the form of or be formulated as) shampoo, shower gel, hand-wash, body-wash or detergent. In accordance with these embodiments of the invention, the invention provides a decontaminant shampoo, shower gel, hand-wash, body-wash or detergent, such as a polymeric adsorbent shampoo, shower gel, hand-wash, body-wash or detergent. The decontaminant shampoo, shower gel, hand-wash, body-wash or detergent comprises the polymeric material defined in the claims (eg. dispersed within the shampoo, shower gel, hand-wash, body-wash or detergent).

In an alternative embodiment, the decontaminant product comprises or consists of (or is in the form of) a flexible material (ie. a material that can be moulded into a range of shapes and sizes, and may be malleable or bendable).

Examples of flexible materials envisaged for the decontaminant product include matrix-based materials such as sponges, or fabric products such as cloths, wipes, towels or flannels.

In one embodiment, the decontaminant product of the invention (as defined in the claims) comprises or consists of (or is in the form of) a sponge, or a fabric product suitable for personal use, such as a cloth, wipe, towel or flannel. In one embodiment, the decontaminant product comprises or consists of adsorbent polymeric material in the form of a sponge or a fabric product suitable for personal use, such as a cloth, wipe, towel or flannel. In one embodiment, the decontaminant product comprises or consists of (or is in the form of) a sponge suitable for personal use. In accordance with this embodiment, there is provided a decontaminant sponge, such as a polymeric adsorbent sponge, which comprises (or consists of) the polymeric material defined in the claims. As used herein, the term "sponge" relates to a porous, matrix-based material that is not limited to any particular porosity or density, and may be produced in any shape or size. In one embodiment, the sponge is small and/ or portable (as defined herein) and is therefore particularly well suited for personal or field use - eg. for decontamination of the human or animal body (such as skin and/or hair) and/ or for decontamination of personal items.

In one embodiment, the decontaminant product of the invention comprises or consists of (or is in the form of) a porous polymeric material. Polymeric material may be made porous by preparation in the presence of a pore-forming agent, such as NaCl. Porosity is particularly advantageous in the 'sponge' aspects of the invention, and thus the invention provides a decontaminant product or polymeric adsorbent as defined herein that comprises or consists of porous polymeric material in the form of a sponge.

In one embodiment, the decontaminant product of the invention, in the form of a sponge or a fabric product suitable for personal use such as a cloth, wipe, towel or flannel, further comprises a transition metal such as zirconium, titanium, manganese, magnesium, calcium, or aluminium (or a salt thereof, eg. an oxide). For example, the sponge or the fabric product such as a cloth, wipe, towel or flannel may further comprise zirconium, or a zirconium salt, such as zirconium oxide.

In one embodiment, the invention provides a decontaminant product of the invention in the form of a sponge, as defined in the claims, wherein said decontaminant product or polymeric adsorbent further comprises a transition metal such as zirconium, titanium, manganese, magnesium, calcium, or aluminium, (or a salt thereof, eg. an oxide). For example, the sponge may further comprise zirconium, or a zirconium salt, such as zirconium oxide. The addition of zirconium or a zirconium salt, such as zirconium oxide, to the sponge increases decontamination efficacy by providing additional chelating capacity.

Alternative product formulations may be used in combination. For example, in one embodiment, the invention provides a decontaminant sponge of the invention (as defined herein) combined with (eg. saturated with) a decontaminant gel or lotion of the invention (as defined herein).

There are a number of important differences to note between the sponge decontaminant product of the present invention (optionally saturated with a gel decontaminant) and the RSDL-saturated sponge used in the art. In this regard, the active component of the decontaminant product (eg. sponge) of the invention is the polymeric material from which it is formed. Thus, the active agent is an intrinsic component of the decontaminant product of the invention, and the decontaminant product (eg. sponge) of the invention can exert its decontaminant effect without the need for an additional decontaminant formulation. By comparison, the RSDL sponge is merely a conventional sponge saturated with the RSDL lotion. As such, the efficacy of the RSDL-saturated sponge depends on the presence of the RSDL lotion, and will vary with the amount of lotion present, and may have a limited shelf life.

A further advantage of the decontaminant sponge (or fabric product) of the present invention (as compared with the RSDL-saturated sponge) is that the decontaminant sponge (or fabric product) of the present invention does not leave a slippery residue behind after use.

Further advantages associated with the 'non-powder' aspects of the invention (ie. sponges, fabric products, and gels etc.) are that the decontaminant product of the invention does not pose an inhalation safety hazard, nor is it associated with the technical problems associated with application/ containment of powdered/ granular decontaminants such as Fullers' Earth.

All embodiments of the decontaminant product of the present invention as defined in the claims apply equally to decontamination kits comprising the decontaminant product of the invention.

All embodiments of the decontaminant product of the present invention as defined in the claims apply equally to decontamination methods and uses employing the decontaminant product.

The present invention provides a decontamination kit comprising (or consisting of) a decontaminant product of the invention as defined in the claims and a sealable and impermeable container (eg. a bag or pouch) for disposal of the decontaminant product following use.

The purpose of the sealable and impermeable container (eg. bag or pouch) is to contain the decontaminant product following use, when it may be contaminated with a hazardous agent. This is particularly useful for preventing off-gassing of contaminants. The sealable and impermeable container may also be used to store the used decontaminant product so that identification and exposure assessment may be performed retrospectively. Assessment may include calculating an estimated dose of hazardous agent. The decontaminant product can be placed in the container following use, and then the container can be sealed and disposed of safely.

In one embodiment, the decontamination kit may further comprise at least one other decontaminant product (eg. Fuller's Earth, a decontaminant lotion such as RSDL lotion, and/ or an RSDL kit comprising a dry wipe and an RSDL-saturated sponge).

The decontamination kit may further comprise a set of instructions for use and/ or an applicator for applying the decontamination product to the surface or object to be decontaminated. The kit may optionally comprise one or more other items useful for decontamination, such as a towel or flannel, hairbrush/ comb, toothbrush, washing products such as soap, shampoo or detergent, protective clothing (eg. protective gloves, mask, glasses or coat) and/ or ventilator equipment.

The quantities and nature of the kit components provided may vary depending on its intended use. For example, the decontamination kit of the invention may be for decontamination of the human or animal body, such as skin, hair or teeth; items of clothing; military equipment (such as armour, weapons or ammunition); laboratory equipment (such as laboratory benches, centrifuges, sinks); medical equipment (such as medical instruments and apparatus, and also hospital furnishings and floors); vehicles (such as aeroplanes, trains, cars or buses); or any part of a public area (including buildings such as airports, stations, schools, arenas).

The decontamination kit is preferably small and compact. In one embodiment, the decontamination kit is a portable decontamination kit. In embodiments of the invention, the decontamination kit is of a size that fits in the human hand and/or can easily be carried by hand, in a pocket, or in a back-pack/ rucksack, and may thus advantageously be suited to field use.

In one embodiment, the kit is for decontamination of a single individual, such as for personal use (ie. self-decontamination of the body, clothing and/ or personal items/ equipment) or for decontamination of another individual. In alternative embodiments, the kit is for decontamination of a group of individuals, or for decontamination of large populations (eg. at a mass gathering such as a sporting event); or for decontamination of one or more items of equipment; or for decontamination of a large area, containing numerous surfaces and objects as described herein.

In one embodiment, the kit components are contained (eg. separately) within suitable packaging material (eg. a container or bag), which may provide a sterile environment for the kit components. In one embodiment, the packaging material containing the kit components is the same as the sealable and impermeable container into which the decontaminant product can be placed following use. Thus, the sealable and impermeable container may be (or may be formed from) the packaging material.

The present disclosure also provides a method for producing a decontaminant product, such as a polymeric adsorbent, as described herein.

The method of the present disclosure comprises forming polymeric material comprising a polymer and formulating the polymeric material as a decontaminant product; wherein forming the polymeric material comprises polymerisation of:

> (i) itaconic acid or a derivative thereof;
> (ii) an acrylate monomer;
> (iii) urocanic acid or a derivative thereof;
> (iv) a vinyl monomer; or
> (v) an amine monomer.

The monomers and derivatives are as defined herein with respect to the decontaminant.

Thus, in one embodiment, the method of the present disclosure comprises forming polymeric material comprising a polymer and formulating the polymeric material as a decontaminant product; wherein forming the polymeric material comprises polymerisation of:

> (i) itaconic acid, or a derivative thereof such as an itaconic ester or an itaconic acid isomer, such as mesaconic acid or citraconic acid;
> (ii) an acrylate monomer selected from the group consisting of 2-trifluoromethyl acrylic acid (TFMAA), methacrylic acid, *N,N*-methylene bisacrylamide, 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, acrolein, or a derivative thereof;
> (iii) urocanic acid, or a derivative thereof such as an ester thereof, such as urocanic acid ethyl ester;
> (iv) a vinyl monomer selected from 1-Vinylimidazole, $_p$-divinylbenzene, $_m$-Divinylbenzene, 2-Vinylpyridine, 4-Vinylpyridine; or
> (v) allylamine.

In one embodiment, forming the polymeric material comprises polymerisation of itaconic acid, 2-trifluoromethyl acrylic acid, *N,N*-methylene bisacrylamide, methacrylic acid or urocanic acid. In accordance with this embodiment, the resulting polymeric material comprises a polymer of itaconic acid, 2-trifluoromethyl acrylic acid, *N,N*-methylene bisacrylamide, methacrylic acid or urocanic acid. For example, the method may comprise polymerisation of itaconic acid or 2-trifluoromethyl acrylic acid, to form polymeric material comprising an itaconic acid polymer or a TFMAA polymer. In one embodiment, the method comprises polymerisation of itaconic acid to form polymeric material comprising an itaconic acid polymer. Alternatively, the method comprises polymerisation of 2-trifluoromethyl acrylic acid to form polymeric material comprising a 2-trifluoromethyl acrylic acid polymer.

As discussed above with respect to the decontaminant product of the invention, the term 'polymer' embraces both homopolymers and co-polymers.

In one embodiment, all the monomers that are polymerised are the same, resulting in the formation of homopolymers. For example, the method may comprise polymerisation of itaconic acid monomers to form an itaconic acid homopolymer.

In an alternative embodiment, at least two (eg. at least 2, 3, 4 or 5) different monomers are polymerised, resulting in the formation of co-polymers. For example, the method may comprise polymerisation of itaconic acid monomers and at least one different monomer to form an itaconic acid co-polymer.

In one embodiment, the polymerising step comprises incubating monomers with a solvent and a free radical initiator. Suitable solvents for use in polymerisation reactions are known in the art, and include dimethylformamide. Suitable free-radical initiators are known in the art, and include 1,1'-azobis(cyclohexanecarbonitrile).

Conventional polymerisation techniques, reagents and apparatus known in the art may be used for the polymerisation step.

The monomers may be polymerised to form linear polymers (eg. a linear IA polymer).

Alternatively (or in addition) the monomers may be polymerised to form cross-linked polymers (eg. a cross-linked IA polymer). In one embodiment, to produce cross-linked polymers, the polymerising step comprises mixing the monomers with a cross-linking agent. Suitable cross-linking agents are known in the art, and include ethylene glycol dimethacrylate (EGDMA).

The ratio of cross-linker to monomer may be in the range of 10:1 to 1:2, such as 8:1, 6:1, 5:1, 4:1, 3:1, 2:1, 1:1, or 1:2. For example, the cross-linker and monomer may be added at a ratio of about 4:1. The inventors have identified that a cross-linker:monomer ratio in this range is particularly suitable for preparing cross-linked powder/ granular decontaminant products of the invention.

The ratio of cross-linker to monomer may alternatively be in the range of 3:1 to 1:2, such as 2.5:1, 2:1, 1.8:1, 1.6:1, 1.5:1, 1.4:1, 1.3:1, 1.2:1, 1:1, 1:1,2 or 1:1.5. For example, the cross-linker and monomer may be added at a ratio of about 1.4:1. The inventors have identified that a cross-linker:monomer ratio in this range is particularly suitable for preparing cross-linked gel decontaminant products of the invention.

Optionally, a transition metal may be added to the reaction mixture, together with the monomers, solvent and free-radical initiator. The transition metal may be in the form of a salt (eg. an oxide), as discussed above with respect to the decontaminant product. In one embodiment, zirconium (eg. a zirconium salt such as $ZrO_2$) is added to the reaction mixture. The inventors have identified that the addition of zirconium is particularly useful for preparing a highly effective itaconic acid-based decontaminant product (eg. an IA-based decontaminant sponge). The addition of zirconium or a zirconium salt, such as zirconium oxide, increases the decontamination efficacy of the decontaminant product by providing additional chelating capacity.

In one embodiment, forming the polymeric material further comprises combining the polymer described above with one or more additional polymers. In an alternative embodiment, forming the polymeric material comprises polymerising the monomers described above in the presence of one or more additional polymers. In accordance with these embodiments, the method of forming the polymeric material may further comprise the initial step of polymerising one or more (eg. 2, 3, 4 or 5) additional monomers to form the one or more additional polymers.

As discussed above with respect to the decontaminant product, the additional polymer(s) may be different from the polymers described above - ie. the polymeric material may be heterogeneous with respect to its polymer content. By way of example, the method may comprise polymerising a monomer (eg. itaconic acid) to form a polymer and combining this polymer with one or more different polymers to form the heterogeneous polymeric material. Alternatively, the method may comprise polymerising a monomer (eg. itaconic acid) in the presence of one or more polymers made from different monomers to form the heterogeneous polymeric material. In one embodiment, the method may comprise attaching (eg. grafting or cross-linking) a polymer onto a backbone comprising one or more different polymers. For example, the method may comprise attaching (eg. grafting or cross-linking) a polymer of the invention (eg. an IA polymer) onto a polymeric backbone comprising PEG.

In one embodiment, the method of the present disclosure is used to produce a polymeric decontaminant in the form of a powder. In accordance with this aspect of the disclosure, the polymeric material (eg. comprising a cross-linked polymer) is ground to a powder having a particle size in the range 10-150µm, such as from at least about 10, 15, 20, 25, 30, 35, 40, 45, 50, 55 or 60 µm, such as up to about 70, 75, 80, 85, 90, 95, 100, 105, 110, 115, 120, 130, 140 or 150 µm. For example, the particle size of the powder may be from about 40 µm to about 90 µm. The polymeric powder may, optionally, subsequently be granulated to form a granular decontaminant. In one embodiment of the method of the disclosure, the polymeric decontaminant is not in the form of (does not comprise or consist of) powder or granules.

In one embodiment, the method comprises formulating the polymer as a sponge, as a gel, liquid, paste, ointment, lotion, cream or foam, or as a fabric product such as a cloth, wipe, towel or flannel.

In one embodiment, the method of the present disclosure is used to produce the polymeric decontaminant in the form of a liquid, gel, paste, ointment, lotion, cream or foam, such as in the form of a gel. In accordance with this aspect of the disclosure, after polymerisation, the polymeric material (eg. comprising one or more linear polymers and/or one or more cross-linked polymers) is separated from the polymerisation mixture (eg. by precipitation), optionally filtered, and formulated in solution, or as a gel, liquid, lotion, ointment, paste or foam formulation. Conventional gel, liquid, lotion, paste and foam formulations are known in the art. In one embodiment, the polymeric material is formulated (eg. dispersed) in a gel formulation. By way of example, the polymeric material may be dispersed in polyethylene glycol (PEG) such as PEG400. Suitable gel formulations may include the polymers at a concentration of 0.1g-0.5g polymer/ml PEG, such as from 0.1, 0.15, 0.2, 0.25, 0.3, 0.4 or 0.5g polymer/ ml PEG, such as at a concentration of about 0.23g polymer/ml PEG.

In one embodiment, the method of the present disclosure is used to produce the polymeric decontaminant in the form of a sponge. In accordance with this aspect of the disclosure, the polymerisation step is typically performed in the presence of a pore-forming agent, such as NaCl. The resulting polymer has a sponge-like consistency, and can be formed (eg. cut) into the desired shape and size. In one embodiment, the method of producing a sponge decontaminant of the invention comprises polymerising any of the monomers described herein in the presence of at least one additional polymer. In accordance with this embodiment, the resultant sponge decontaminant product comprises polymeric material comprising the polymers defined herein attached to (eg. cross-linked with or grafted onto) one or more additional polymers. By way of example, a method of producing a sponge decontaminant of the invention may comprise polymerising itaconic acid in the presence of polyethylene glycol (PEG), resulting in polymeric material comprising itaconic acid polymer attached to (eg. cross-linked with or grafted onto) a PEG backbone.

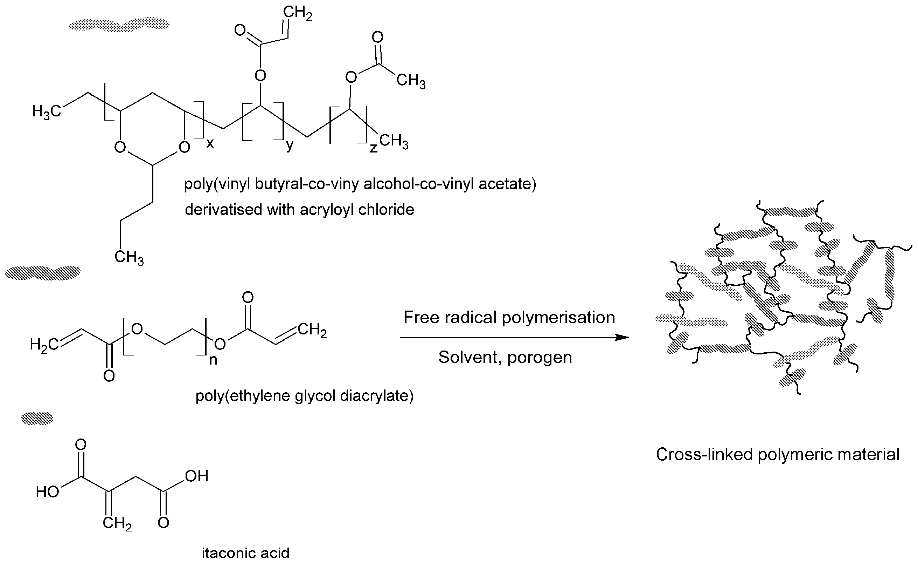

The inventors have achieved good results by polymerising monomers (eg. itaconic acid) in the presence of (i) poly(vinyl butyral-co-vinyl acetate) (PBAA) derivatised with acryloyl chloride and (ii) polyethylene glycol (PEG)-diacrylate. This method results in polymeric material comprising itaconic acid polymers cross-linked to a polymeric 'backbone' comprising PEG. This method is illustrated in Figure 1.

In one embodiment, the method of the present disclosure is used to produce the decontaminant product of the invention in the form of a fabric product such as a cloth, wipe, towel or flannel. In accordance with this aspect of the disclosure, after polymerisation, the polymers may be electrospun into fibres. The electrospun polymer fibres may then be woven to produce a fabric product, or may alternatively be attached to the fibres of a pre-formed fabric product.

The disclosure further provides the use of a polymer for the manufacture of a decontaminant product or polymeric adsorbent, as defined herein, wherein the polymer is a polymer of:

(i) itaconic acid or a derivative thereof;
(ii) an acrylate monomer;
(iii) urocanic acid or a derivative thereof;
(iv) a vinyl monomer; or
(v) an amine monomer.

The monomers and derivatives thereof are as defined herein with respect to the decontaminant product (see above).

Thus, in one embodiment, the disclosure further provides the use of a polymer for the manufacture of a decontaminant product or polymeric adsorbent, as defined herein, wherein the polymer is a polymer of:

(i) itaconic acid, or a derivative thereof such as an itaconic ester or an itaconic acid isomer, such as mesaconic acid or citraconic acid;
(ii) an acrylate monomer selected from the group consisting of 2-trifluoromethyl acrylic acid (TFMAA), methacrylic acid, *N,N*-methylene bisacrylamide, 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, acrolein, or a derivative thereof;
(iii) urocanic acid, or a derivative thereof such as an ester thereof, such as urocanic acid ethyl ester;
(iv) a vinyl monomer selected from 1-Vinylimidazole, $_p$-divinylbenzene, $_m$-Divinylbenzene, 2-Vinylpyridine, 4-Vinylpyridine; or
(v) allylamine.

In one embodiment, the polymer is a polymer of itaconic acid (IA), 2-trifluoromethyl acrylic acid (TFMAA), methacrylic acid, *N,N*-methylene bisacrylamide or urocanic acid. In one embodiment, the polymer is a polymer of itaconic acid or of 2-trifluoromethyl acrylic acid. In one embodiment, the polymer is an itaconic acid polymer. In an alternative embodiment, the polymer is 2-trifluoromethyl acrylic acid.

The polymer may be for the manufacture of any embodiment of the decontaminant product of the invention, such as a polymeric adsorbent, as defined herein.

The disclosure also provides the use of a polymer for decontaminating a surface or object that is contaminated, or suspected to be contaminated, with a hazardous agent (typically a hazardous chemical agent); wherein the polymer is a polymer of:

(i) itaconic acid or a derivative thereof;
(ii) an acrylate monomer;
(iii) urocanic acid or a derivative thereof;
(iv) a vinyl monomer; or
(v) an amine monomer.

The monomers and derivatives thereof are as defined herein with respect to the decontaminant product (see above).

Thus, in one embodiment, the disclosure provides the use of a polymer for decontaminating a surface or object that is contaminated, or suspected to be contaminated, with a hazardous agent (eg. a hazardous chemical agent such as a chemical warfare agent); wherein the polymer is a polymer of:

(i) itaconic acid, or a derivative thereof such as an itaconic ester or an itaconic acid isomer, such as mesaconic acid or citraconic acid;
(ii) an acrylate monomer selected from the group consisting of 2-trifluoromethyl acrylic acid (TFMAA), methacrylic acid, *N,N*-methylene bisacrylamide, 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, acrolein, or a derivative thereof;
(iii) urocanic acid, or a derivative thereof such as an ester thereof, such as urocanic acid ethyl ester;
(iv) a vinyl monomer selected from 1-Vinylimidazole, $_p$-divinylbenzene, $_m$-Divinylbenzene, 2-Vinylpyridine, 4-Vinylpyridine; or
(v) allylamine.

The disclosure provides the use of a polymer as an adsorbent for decontaminating a surface or object that is contaminated, or suspected to be contaminated, with a hazardous agent (typically a hazardous chemical agent); wherein the polymer is a polymer of:

(i) itaconic acid or a derivative thereof;
(ii) an acrylate monomer;
(iii) urocanic acid or a derivative thereof;
(iv) a vinyl monomer; or
(v) an amine monomer.

The monomers and derivatives thereof are as defined herein with respect to the decontaminant product (see above).

Thus, in one embodiment, the disclosure provides the use of a polymer as an adsorbent for decontaminating a surface or object that is contaminated, or suspected to be contaminated, with a hazardous agent (eg. a hazardous chemical agent such as a chemical warfare agent); wherein the polymer is a polymer of:

(i) itaconic acid, or a derivative thereof such as an itaconic ester or an itaconic acid isomer, such as mesaconic acid or citraconic acid;
(ii) an acrylate monomer selected from the group consisting of 2-trifluoromethyl acrylic acid (TFMAA), methacrylic acid, *N,N*-methylene bisacrylamide, 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, acrolein, or a derivative thereof;
(iii) urocanic acid, or a derivative thereof such as an ester thereof, such as urocanic acid ethyl ester;
(iv) a vinyl monomer selected from 1-Vinylimidazole, $_p$-divinylbenzene, $_m$-Divinylbenzene, 2-Vinylpyridine, 4-Vinylpyridine; or
(v) allylamine.

The invention further provides the use of a decontaminant product of the invention (as defined in the claims) for decontaminating a surface or object that is contaminated, or suspected to be contaminated, with a hazardous chemical agent (eg. typically a chemical warfare agent), wherein the surface or object is any part of the human or animal body, such as skin, hair or teeth; clothing; or personal items or equipment.

The present invention further provides a method of decontaminating an object or surface that is contaminated, or suspected to be contaminated, with a hazardous chemical agent; comprising contacting the object or surface with a decontaminant product of the invention as defined in the claims, wherein the surface or object is any part of the human or animal body, such as skin, hair or teeth; clothing; or personal items or equipment. As used herein, the term "decontaminating" (or related terms such as "decontamination") means at least partially removing, detoxifying or neutralising a potentially hazardous agent (eg. hazardous chemical agent), thereby reducing or eliminating potential harm or damage caused by exposure to the agent. In one embodiment, the term "decontaminating" (or related terms such as "decontamination") means removing, or at least partially removing, a potentially hazardous agent (eg. a hazardous chemical agent, such as a CWA) from a surface or object, thereby reducing or eliminating potential harm or damage caused by exposure of the surface or object to the agent.

Thus, in accordance with this aspect of the invention, the use or method at least partially removes, detoxifies or neutralises the potentially hazardous chemical agent thereby reducing or eliminating potential harm or damage caused by exposure to the agent. Thus, following use or the method of the invention, the potentially hazardous chemical agent has been at least partially removed, detoxified or neutralised, thereby reducing or eliminating potential harm or damage caused by exposure to the agent. In one embodiment, the use or method of the invention removes, detoxifies, or neutralises at least 5% (eg. at least 10, 15, 20, 25, 30, 35, 40, 45, 50, 55, 60, 65, 70, 75, 80, 85, 90, 95 or 100%) of the potentially hazardous chemical agent. For example, the use or method of the invention removes, detoxifies, or neutralises at least 60% (eg. at least 65, 70, 75, 80, 85, 90, 95 or 100%) of the potentially hazardous chemical agent.

In one embodiment, the use or method removes, or at least partially removes, the potentially hazardous chemical agent (such as a CWA) from a surface or object, thereby reducing or eliminating potential harm or damage caused by exposure to the agent. Thus, following use or the method of the invention, the potentially hazardous chemical agent has been removed, or at least partially removed, from the surface or object, thereby reducing or eliminating potential harm or damage caused by exposure of the surface or object to the agent. In one embodiment, the use or method of the invention removes at least 5% (eg. at least 10, 15, 20, 25, 30, 35, 40, 45, 50, 55, 60, 65, 70, 75, 80, 85, 90, 95 or 100%) of the potentially hazardous chemical agent from the surface or object. For example, the use or method of the invention removes at least 60% (eg. at least 65, 70, 75, 80, 85, 90, 95 or 100%) of the potentially hazardous chemical agent from the surface or object.

In one embodiment, the removal (or at least partial removal) of the agent from the surface or object is adsorption of the agent by the polymeric material. Thus, following use or the method of the invention, the potentially hazardous chemical agent has been adsorbed, or at least partially adsorbed, from the surface or object.

In one embodiment, the use or method of the invention adsorbs at least 5% (eg. at least 10, 15, 20, 25, 30, 35, 40, 45, 50, 55, 60, 65, 70, 75, 80, 85, 90, 95 or 100%) of the agent from the surface or object. For example, the use or method of the invention adsorbs at least 60% (eg. at least 65, 70, 75, 80, 85, 90, 95 or 100%) of the agent from the surface or object.

In one embodiment, the use or method reduces the potential harm or damage caused by exposure to the agent by at least 5% (eg. by at least 10, 15, 20, 25, 30, 35, 40, 45, 50, 55, 60, 65, 70, 75, 80, 85, 90, 95 or 100%). In one embodiment, the contamination remaining following the use or method of the invention less than 95% (eg. less than 90, 85, 80, 75, 70, 65, 60, 55, 50, 45, 40, 35, 30, 25, 20, 15, 10 or 5%) of the contamination present prior to carrying out the use or method.

The level of % decontamination achieved with the present invention is typically assessed by exposing a defined surface or object (eg. skin, typically a test skin sample such as a sample of animal skin, eg. pig skin) to a defined amount of contaminating agent, contacting the contaminated surface/ object with the decontaminant product of the invention, and measuring the amount of contaminant retained into or bound to the decontaminant product or polymeric adsorbent from the surface. Alternatively, % decontamination could be assessed by measuring the amount of contaminant remaining on the surface or object following contact with the decontaminant product of the invention. It is understood that the % level decontamination achieved may vary depending on the nature of the contaminating agent, the nature of the contaminated surface, and the type of polymer(s) present in the decontamination product. In addition, the % level of decontamination may decrease the longer that the contaminating agent is left in contact with the surface prior to decontamination. For example, in one embodiment, an 85-90% efficiency of decontamination can be achieved with a decontaminant product of the invention when a skin sample is exposed to a contaminating agent for 5 minutes. However, the efficiency of decontamination drops to 45-65% following 60 minutes of exposure.

Thus, in one embodiment, the use or method of the invention is performed within 60 minutes of the surface or object being contaminated with the hazardous chemical agent, preferably within 40, 30, 20, 15, 20 or 5 minutes from the time of contamination.

As used herein, the term 'contacting' embraces, but is not limited to, methods of bringing together the decontaminant product with the contaminated object or surface by any means, such as by dabbing, wiping, rubbing, scrubbing, spraying, brushing, soaking, pressing, compressing, coating or wrapping. A skilled person will understand that the contacting technique to be used will vary with the object or surface to be decontaminated, and with the contaminating agent, and is able to select an appropriate technique depending on the circumstances. By way of example, the decontaminant sponge of the invention may be wiped, rubbed, scrubbed or pressed against a contaminated surface or object (eg. skin, clothing, equipment). By way of example, the decontaminant gel of the invention may be contacted with a surface or object (eg. skin, clothing, equipment) by dabbing, wiping, rubbing, scrubbing, smearing etc., optionally in combination with water.

Likewise, a skilled person will understand that the duration of contacting will vary with the circumstances, for example, with the type or amount of hazardous agent present or suspected to be present and/ or with the object or surface to be contacted. For example, adequate decontamination may be achieved by contacting the decontaminant with the surface or object for a few seconds (eg. about 5-10 seconds). Alternatively, the decontaminant may be contacted with the surface or object for at least 30 seconds, or at least 1, 2, 5 or 10 minutes, or for at least half an hour, or for at least one hour. In some circumstances, adequate decontamination (eg. of large amounts of hazardous agent) may require contacting the surface or object with the decontaminant product for longer periods, such as at least 2, 4, 6, 12 or 24 hours. However, if the contaminated surface is skin, the decontaminant sponge is typically contacted with the skin for up to about 2 hours (eg. for less than 2 hours, or less than 1 hour), to minimise skin occlusion effects.

In one embodiment, the object or surface is known to be contaminated with a hazardous chemical agent as defined herein. In another embodiment, the object or surface is suspected to be contaminated with a hazardous chemical agent as defined herein.

The use or method of the invention can be for decontamination of any of the hazardous chemical agents described herein. In one embodiment, the use or method is for decontamination of chemical agents such as chemical warfare agents and surrogates thereof. The Examples of the present application demonstrate the utility of the invention for decontaminating surfaces or objects that are contaminated with methyl salicylate, sulphur mustard, VX or soman.

The use or method of the invention is for decontamination of any of the surfaces or objects defined in the claims. In one embodiment, the use or method is for decontamination of human skin or hair.

The invention provides a barrier product for protecting against contamination of a surface or object by a hazardous chemical agent, wherein the protective barrier product comprises a protective cover, a protective garment, or a barrier cream, lotion, gel, foam, or paste; and comprises polymeric material comprising a polymer of:

(i) itaconic acid, or a derivative thereof such as an itaconic ester or an itaconic acid isomer, such as mesaconic acid or citraconic acid;
(ii) an acrylate monomer selected from the group consisting of 2-trifluoromethyl acrylic acid (TFMAA), methacrylic acid, *N,N*-methylene bisacrylamide, 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile, acrolein, or a derivative thereof;
(iii) urocanic acid or a derivative thereof, such as an ester thereof, such as urocanic acid ethyl ester;
(iv) a vinyl monomer selected from 1-Vinylimidazole, $_p$-divinylbenzene, $_m$-Divinylbenzene, 2-Vinylpyridine, 4-Vinylpyridine; or
(v) allylamine;

and wherein the surface or object is any part of the human or animal body, such as skin, hair or teeth; clothing; or personal items or equipment.

The monomers and derivatives thereof are as defined herein with respect to the decontaminant product (see above).

The polymer, polymeric material, and methods of forming these barrier products are as described herein with respect to the decontaminant product of the invention. The invention also provides a method of protecting a surface or object from contamination by a hazardous agent, comprising applying a barrier product of the invention to the surface or object; wherein the surface or object is any part of the human or animal body, such as skin, hair or teeth; clothing; or personal items or equipment.

The disclosure further provides the use of a barrier product described herein for protecting a surface or object from contamination by a hazardous agent; and a barrier product described herein for use in protecting a surface or object from contamination by a hazardous agent.

The term 'contamination' is defined herein. As used herein, the term 'protecting a surface or object from contamination by a hazardous agent' embraces reducing or preventing contamination of the surface or object. Thus, in one embodiment, the barrier product, or method of the invention is for reducing or preventing contamination of a surface or object by a hazardous agent. The barrier product and method of the invention may protect against contamination by reducing or preventing the deposition or binding of a hazardous agent to the surface or object, or by reducing or preventing penetration (eg. absorption) of a hazardous agent into the surface or object. For example, the barrier product may form a physical barrier between the hazardous agent and the surface or object. The barrier product and method of the invention may alternatively, or additionally, protect against contamination of the surface or object by capturing, sequestering, destroying, detoxifying, or neutralising the hazardous agent.

The barrier product of the invention is typically applied to the surface or object (as defined in the claims) prior to any contamination (eg. in anticipation of contamination occurring). Alternatively, the barrier product may be applied after contamination has occurred (eg. to reduce or prevent further contamination of the surface or object). Thus, the barrier product of the invention can be used to protect a surface or object (as defined in the claims) from contamination that may (or may not) occur in the future.

Thus, in one embodiment, the method of the invention comprises applying the barrier product to the surface or object (as defined in the claims) before the surface or object is exposed to contamination (eg. if it is identified that the surface or object is at risk of potential contamination in the future). For example, the barrier product may be applied to the surface or object (as defined in the claims) when the surface or object is produced. In another embodiment, the method of the invention comprises applying the barrier product to a surface or object (as defined in the claims) that has been exposed to contamination, in order to protect the surface or object from further potential contamination.

As used herein, the term 'applying a barrier product of the invention to the surface or object' embraces using the barrier product to at least partially (or entirely) cover, wrap or mask the surface or object. The barrier product may take different forms, and will be applied to the surface or object in different ways, and for different durations, depending on the surface or object to be protected.

Surfaces or objects that can be protected using the barrier product of the invention are as defined in the claims, and further described herein with respect to the decontaminant product of the invention. As defined in the claims, the barrier product is for protecting any part of the human or animal body (such as the skin, hair or teeth), or is for protecting clothing, or personal items or equipment (such as any machinery, or vehicle, as described herein). In one embodiment, the barrier product is for protecting human skin or hair from contamination.

In one embodiment, the barrier product of the present invention comprises (or consists of) a cream, lotion, gel, foam or paste. For example, the barrier product may be a barrier cream. In accordance with this embodiment, the cream, lotion, gel, foam or paste comprises the polymeric material defined in the claims. A barrier cream, lotion, gel or paste of the invention may be produced using a method as defined above for the decontaminant cream, lotion, gel or paste of the invention.

Barrier creams, lotions, gels, foams or pastes of the invention can be applied to a surface or object by any means - eg. by spreading, wiping, rubbing, dabbing, or pressing the product onto or into the surface or object. Barrier creams, lotions, gels, foams or pastes of the invention are particularly suitable for protecting skin or hair from contamination. The invention thus provides a method of protecting skin from contamination, comprising applying a barrier cream, lotion, gel, foam or paste of the invention to skin or hair. The invention further provides the use of a barrier cream, lotion, gel, foam or paste of the invention for protecting skin or hair from contamination; and also provides a barrier cream, lotion, gel, foam or paste of the invention for use in protecting skin or hair from contamination.

In one embodiment, the barrier product of the present invention comprises (or consists of) a protective cover. The protective cover comprises the polymeric material defined in the claims. A barrier product comprising or consisting of a protective cover may be produced using a method as defined above for the decontaminant sponge or fabric product. Examples of protective cover embraced by the invention include a bag, tent, protective shelter, or other protective cover such as a cover for equipment/ machinery (eg. military, laboratory or medical equipment) or for a vehicle.

Protective covers of the invention can be applied to a surface or object by any suitable means - eg. by wrapping, enveloping, enclosing or bagging the surface or object. The invention thus provides a method of protecting equipment, machinery or a vehicle from contamination, comprising applying a protective cover of the invention to the equipment, machinery or a vehicle. The disclosure further provides the use of a protective cover described herein for protecting equipment, machinery or a vehicle from contamination; and also provides a protective cover described herein for use in protecting equipment, machinery or a vehicle from contamination.

In one embodiment, the barrier product of the present invention comprises (or consists of) a protective garment. The protective garment of the invention comprises the polymeric material defined in the claims. A barrier product comprising or consisting of a protective garment may be produced using a method as defined above for the decontaminant sponge or fabric product. Protective garments of the invention are particularly suitable for protecting a human or animal, or a part thereof (eg. skin, hair) or clothing. As used herein, the term protective garment embraces, but is not limited to, a gown, coat (eg. lab coat), overalls, or suit (eg. a hazmat suit, a military NBC suit), face or eye mask, gloves, hair-cover, shoe protectors, or any other protective covering that can be applied to any part of the human or animal body.

Individuals can protect themselves from contamination by wearing the protective garment, or can protect other individuals (humans or animals) by putting the protective garment on the other individual or animal so that it is worn by the other individual/ animal. The disclosure thus provides the use of a protective garment described herein for protecting a human or animal from contamination; and also provides a protective garment described herein for use in protecting a human or animal from contamination.

The invention also provides a barrier kit comprising a barrier product of the invention as defined in the claims, and one or more of: (i) a sealable and impermeable container for disposal of the barrier product following use; (ii) a decontaminant product (eg. a decontaminant product as defined herein); (iii) a towel, flannel, hairbrush, comb, toothbrush, soap, shampoo, detergent, protective clothing (eg. protective gloves, mask, glasses or coat) and/ or ventilator equipment; and/ or (iv) a set of instructions for use.

In one embodiment, the level of protection provided by the barrier product of the present invention can be measured by applying the barrier product to a defined surface (eg. skin), applying a defined quantity of an agent to the barrier product, and assessing the amount of a defined agent that penetrates through the barrier product to the skin over time. The invention will be further clarified by the following examples and figures, which are intended to be purely exemplary of the invention and in no way limiting.

Figure 1: Schematic diagram of polymerisation reaction used to generate polymeric material in the form of a sponge.

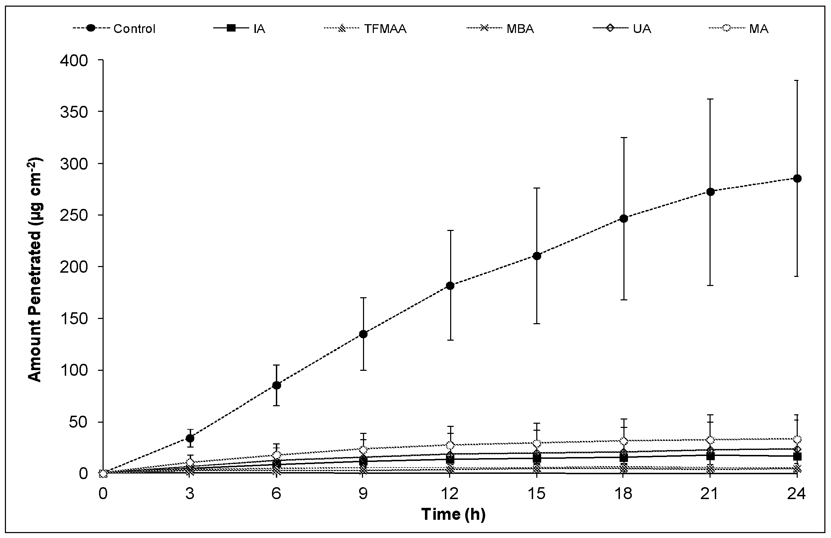

Figure 2: Cumulative amount of $^{14}$C-radiolabelled methyl salicylate penetrating untreated (control) or decontaminated pig skin over a 24 hour period. Skin surface decontamination was conducted five minutes post-exposure using powder formulations of IA, TFMAA, MBA, UA and MA. All points are mean $\pm$ standard deviation of n=6 diffusion cells.

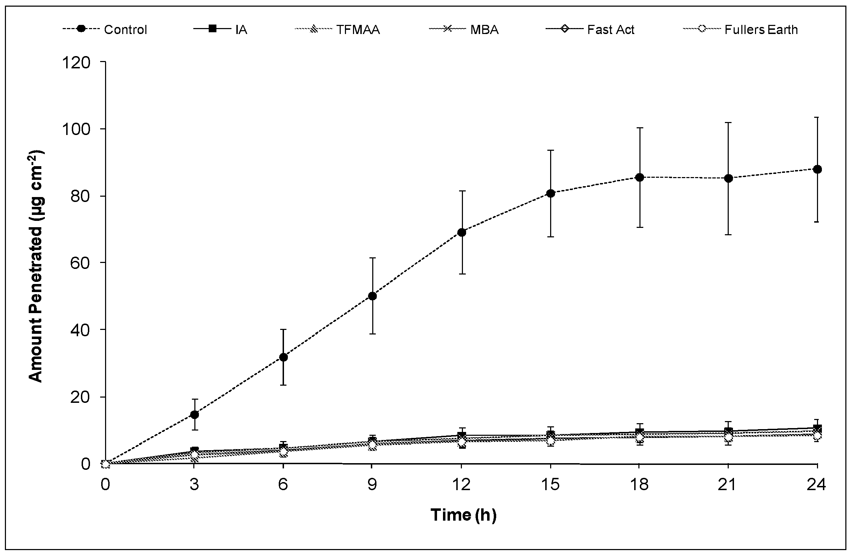

Figure 3: Cumulative amount of $^{14}$C-radiolabelled Sulphur Mustard penetrating untreated (control) or decontaminated pig skin over a 24 hour period. Skin surface decontamination was conducted five minutes post-exposure using powder formulations of IA, TFMAA, MBA, Fast Act and Fullers' Earth. All points are mean $\pm$ standard deviation of n=6 diffusion cells.

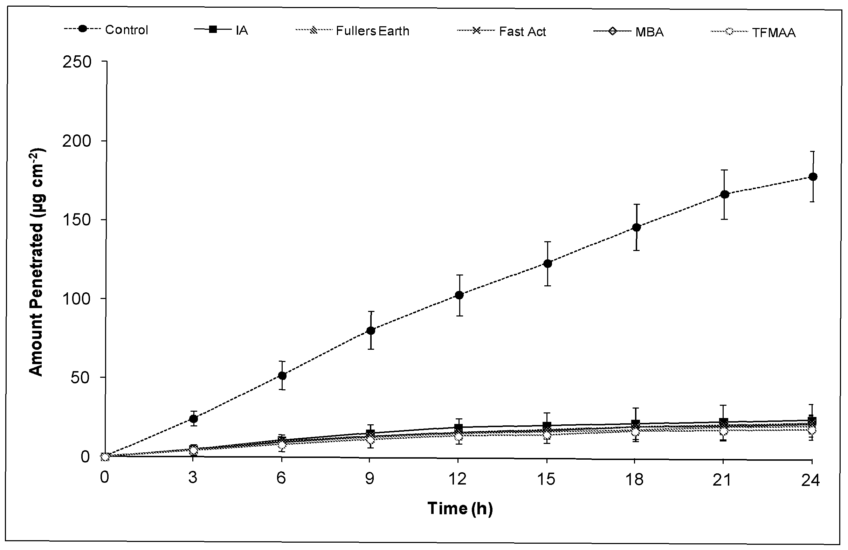

Figure 4: Cumulative amount of $^{14}$C-radiolabelled Soman penetrating untreated (control) or decontaminated pig skin over a 24 hour period. Skin surface decontamination was conducted five minutes post-exposure using powder formulations of IA, TFMAA, MBA, Fast Act and Fullers' Earth. All points are mean $\pm$ standard deviation of n=6 diffusion cells.

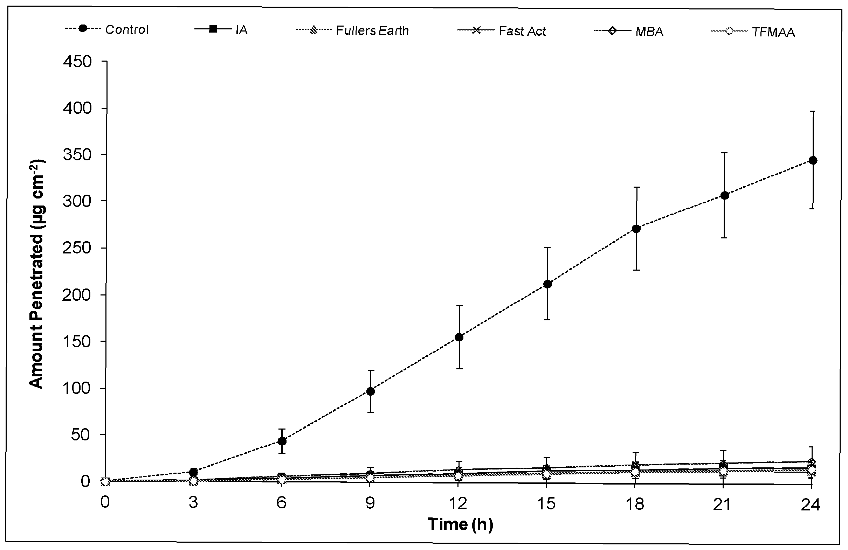

Figure 5: Cumulative amount of $^{14}$C-radiolabelled VX penetrating untreated (control) or decontaminated pig skin over a 24 hour period. Skin surface decontamination was conducted five minutes post-exposure using powder formulations of IA, TFMAA, MBA, Fast Act and Fullers' Earth. All points are mean $\pm$ standard deviation of n=6 diffusion cells.

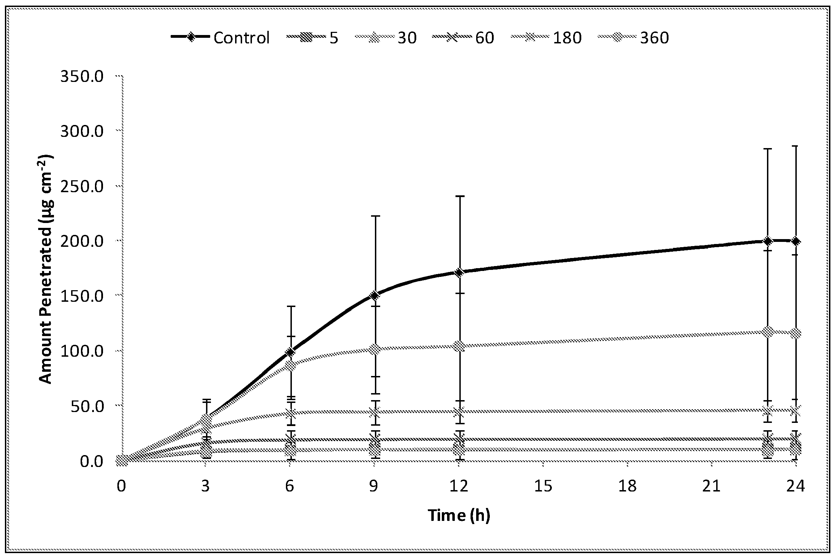

Figure 6: Cumulative amount of $^{14}$C-radiolabelled Methyl salicylate penetrating untreated (control) or decontaminated pig skin over a 24 hour period. Skin surface decontamination was conducted 5, 30, 60, 180 & 360 minutes post-exposure using sponge formulations of TFMAA. All points are mean $\pm$ standard deviation of n=6 diffusion cells.

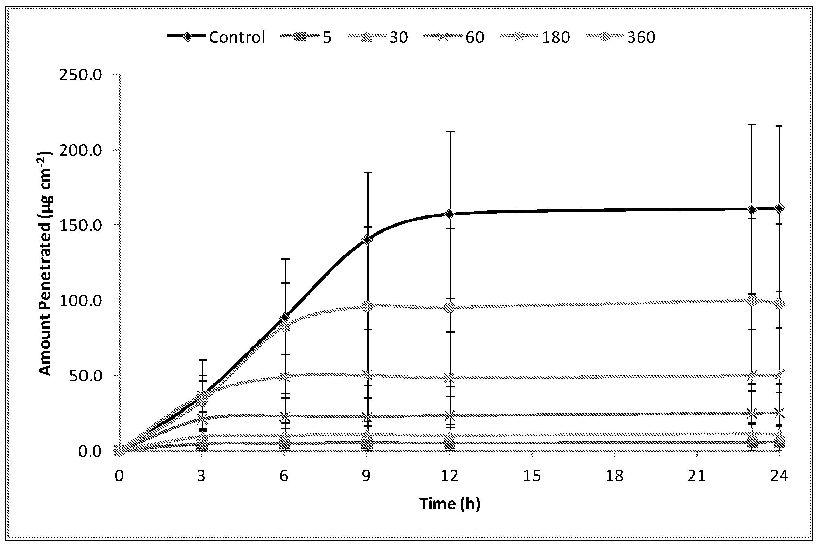

Figure 7: Cumulative amount of $^{14}$C-radiolabelled Methyl Salicylate penetrating untreated (control) or decontaminated pig skin over a 24 hour period. Skin surface decontamination was conducted 5, 30, 60, 180 & 360 minutes post-exposure using sponge formulations of IA. All points are mean $\pm$ standard deviation of n=6 diffusion cells.

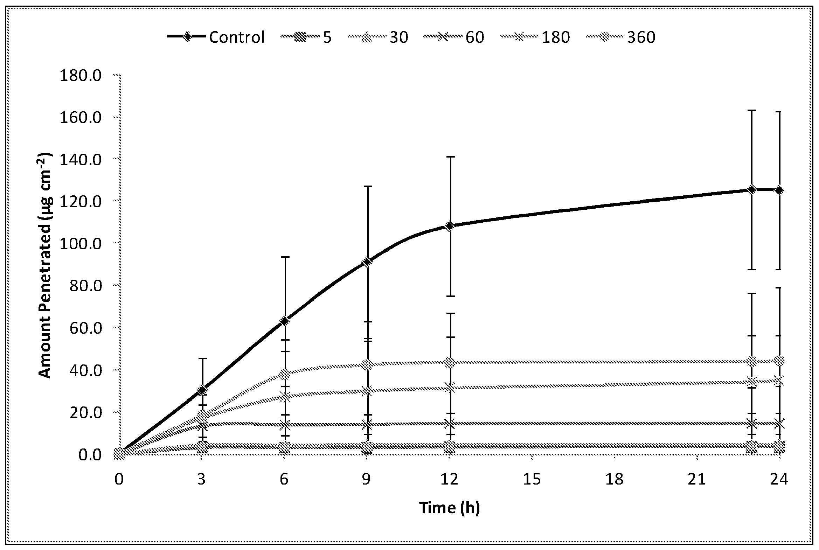

Figure 8: Cumulative amount of $^{14}$C-radiolabelled Sulphur Mustard penetrating untreated (control) or decontaminated pig skin over a 24 hour period. Skin surface decontamination was conducted 5, 30, 60, 180 & 360 minutes post-exposure using sponge formulations of IA. All points are mean $\pm$ standard deviation of n=6 diffusion cells.

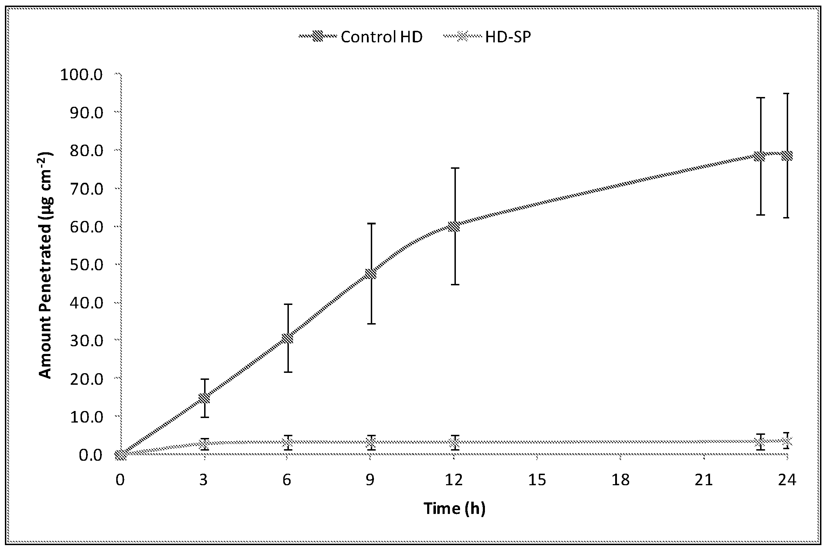

Figure 9: Cumulative amount of $^{14}$C-radiolabelled Sulphur Mustard penetrating untreated control (HD) or decontaminated (HD-SP) pig skin over a 24 hour period. Skin surface decontamination was conducted five minutes post-exposure using sponge formulations of IA with Zirconium. All points are mean $\pm$ standard deviation of n=6 diffusion cells.

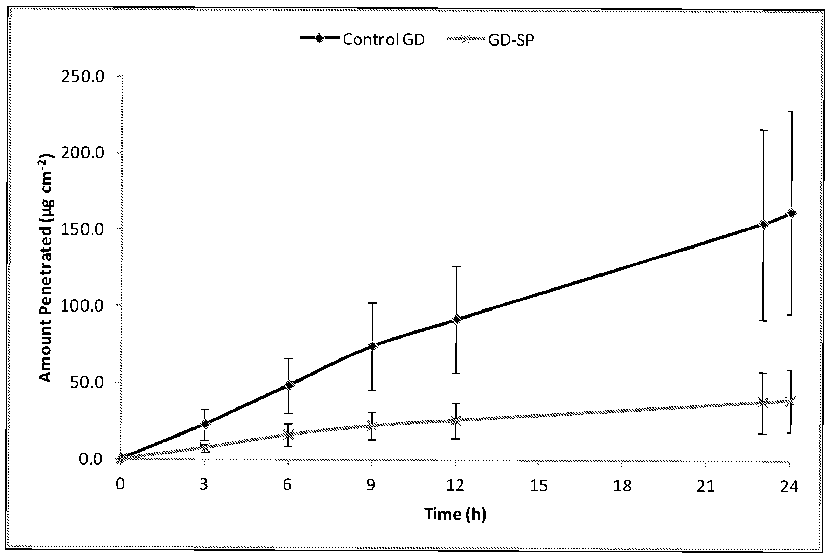

Figure 10: Cumulative amount of $^{14}$C-radiolabelled Soman penetrating untreated control (GD) or decontaminated (GD-SP) pig skin over a 24 hour period. Skin surface decontamination was conducted five minutes post-exposure using sponge formulations of IA with Zirconium. All points are mean $\pm$ standard deviation of n=6 diffusion cells.

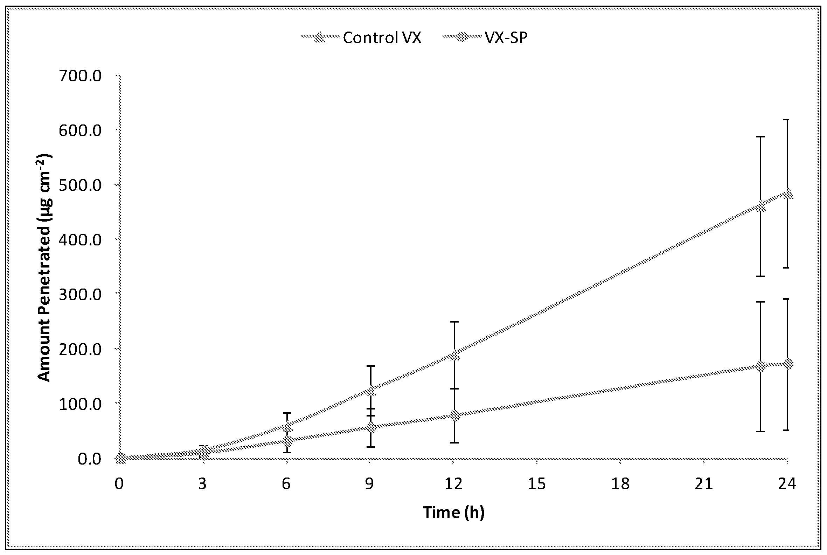

Figure 11: Cumulative amount of $^{14}$C-radiolabelled VX penetrating untreated control (VX) or decontaminated (VX-SP) pig skin over a 24 hour period. Skin surface decontamination was conducted five minutes post-exposure using sponge formulations of IA with Zirconium. All points are mean $\pm$ standard deviation of n=6 diffusion cells.

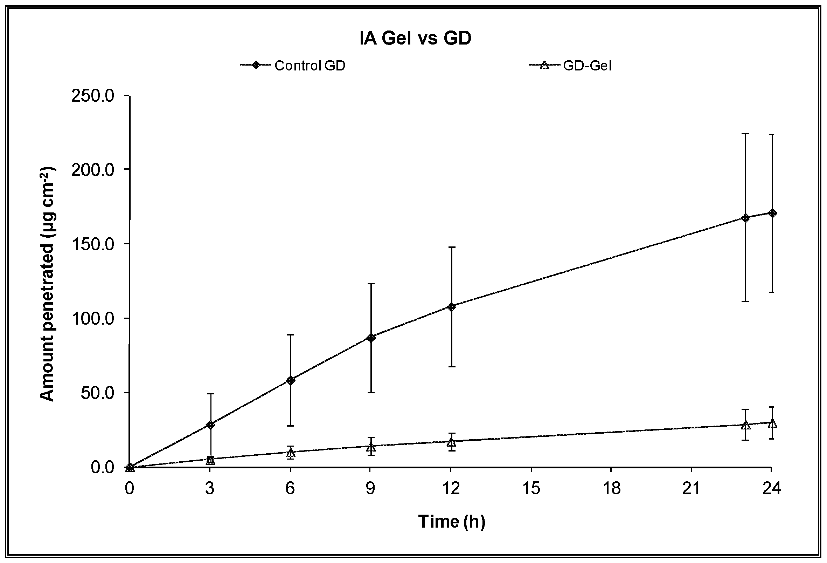

Figure 12: Cumulative amount of $^{14}$C-radiolabelled Soman penetrating untreated control (GD) or decontaminated (GD-gel) pig skin over a 24 hour period. Skin surface decontamination was conducted five minutes post-exposure using Gel formulations of IA. All points are mean ± standard deviation of n=6 diffusion cells.

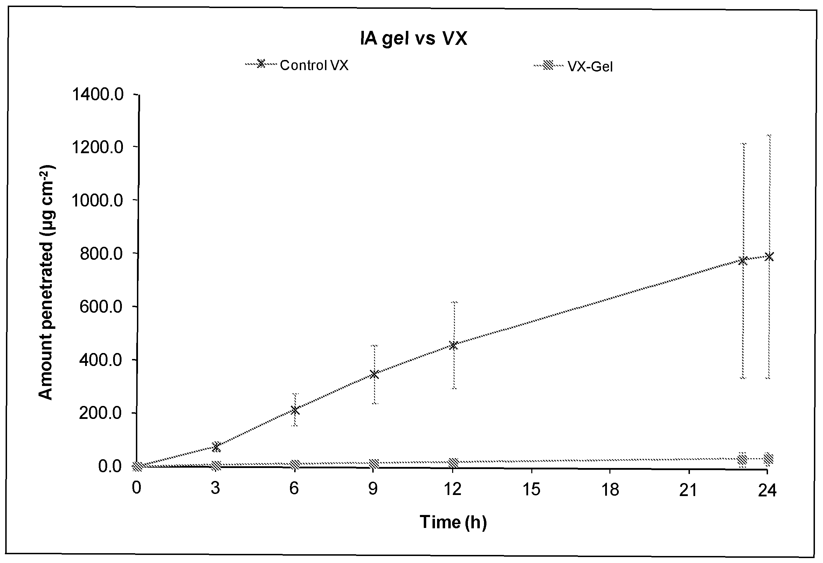

Figure 13: Cumulative amount of $^{14}$C-radiolabelled VX penetrating untreated control (VX) or decontaminated (VX-gel) pig skin over a 24 hour period. Skin surface decontamination was conducted five minutes post-exposure using Gel formulations of IA. All points are mean ± standard deviation of n=6 diffusion cells.

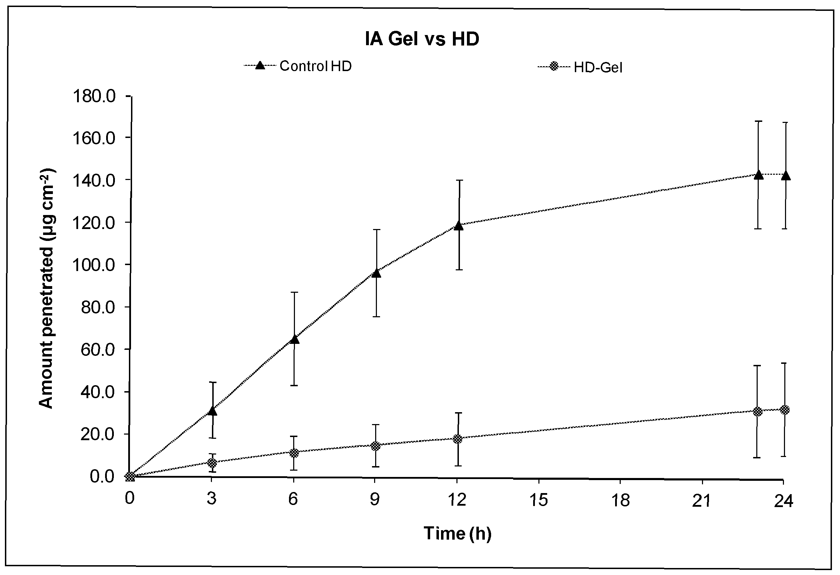

Figure 14: Cumulative amount of $^{14}$C-radiolabelled Sulphur Mustard penetrating untreated control (HD) or decontaminated (HD-gel) pig skin over a 24 hour period. Skin surface decontamination was conducted five minutes post-exposure using Gel formulations of IA. All points are mean ± standard deviation of n=6 diffusion cells.

## EXAMPLES

### Example 1: Identification of monomers

20 functional monomers were identified (Table 1, below), which possess polymerisable residues and residues capable of interacting with an agent through ionic and hydrogen bonds, van der Waal's and dipole-dipole interactions.

Table 1:

| 1-Vinylimidazole | 2-Vinylpiridine | Acrylamide | 2-Hydroxyethyl Methacrylate |
|---|---|---|---|
| $_p$-divinylbenzene | 4-Vinylpiridine | Acrolein | Acrylamido-2-methyl-1-propanesulfonic acid (AMPSA) |
| Acrylic acid | Acrylonitrile | Allylamine | 2-Trifluoromethyl acrylic acid (TFMAA) |
| N,N-Methylene Bis Acrylamide (MBA) | Urocanic acid (UA) | Urocanic Acid Ethyl Ester | N,N-Diethylamino Ethyl Methacrylate (DEAEM) |
| $_m$-Divinylbenzene | Methacrylic acid (MA) | Itaconic Acid (IA) | Ethylene Glycol Dimethacrylate (EGDMA) |

The functional monomers were screened for their ability to form a molecular complex with the agents Dimethyl Sulfoxide, Methyl Salicylate, Parathion, Soman, Sulphur Mustard, VX and tris(3-(2,2,3,3,4,4,4-heptafluorobutyryl)bornane-2-onato-O,O')europium.

The functional monomers were screened and ranked for their ability to form a molecular complex with the agent using the *Leapfrog* algorithm; monomers having the highest binding score (kcal mol$^{-1}$) being the best candidates for polymer preparation. The program was applied for 32,000 iterations in order to fully examine the binding between the monomers with the agent.

The highest-ranking 5 monomers (Itaconic acid (IA), 2-Trifluoromethyl acrylic acid (TFMAA), N-N-Methylene BisAcrylamide (MBA), Urocanic acid (UA) and Methacrylic acid (MA)) were selected for further evaluation against Methyl Salicylate and chemical warfare agents (Soman, Sulphur Mustard and VX).

These 5 polymers are considered further in the following Examples. However, any or all of the monomers listed in Table 1 may be used alone or in combination to produce the decontaminant of the present invention.

### Example 2: Polymer synthesis and composition (IA powder formulation)

Cross-linked IA polymer was synthesised by mixing the cross-linker Ethylene Glycol Dimethacrylate (EGDMA) and IA in a glass bottle at a 4:1 (molar ratio) EDGMA: IA. The mixture was dissolved in the same mass (as that of EGDMA + IA) of the solvent dimethylformamide (DMF), and then 1% (in mass) of the free-radical initiator 1,1'-azobis(cyclohexanecarbonitrile) was added to the solution of monomers and DMF. The monomers, initiator and solvent were mixed and nitrogen bubbled for 5min in order to remove dissolved oxygen from solution and headspace. The bottle was then sealed with a screw cap and the reaction initiated thermally at 80°C and kept at that temperature for 18 hours.

After polymerisation, the polymer was ground and wet-sieved with methanol and particles with size ranging from 40-90μm collected. These were then washed with hot methanol on a soxhlet over 24hours and dried at 80°C overnight.

This method can also be performed using the monomers TFMAA, MBA, UA or MA, or any of the other monomers recited in Table 1.

**Example 2: Polymer synthesis and composition (IA sponge formulation)**

Formulation without Zirconium (see Figure 1):

Flexible cross-linked sponges containing itaconic acid (IA) moieties were synthesised by first derivatising poly(vinyl butyral-co-vinyl acetate) (PBAA) with an average molecular weight of 50.00 - 80.00 with acryloyl chloride. For this purpose 24g PBAA was dissolved in 360ml of anhydrous methlpyrrolidone and 6ml acryloyl chloride add dropwise under stirring. The mixture was then allowed to react under stirring in the dark at 0°C for two hours.

After two hours, 48g of Itaconic acid 60ml poly(ethylene glycol) diacrylate (average $M_n$ 575) and 10.8g free-radial initiator 1,1'-azobis(cyclohexanecarbonitrile) were added to the mixture. In order to create large pores in the final sponge, 2.5Kg NaCl (fine cooking salt grade) were thoroughly mixed with the above mixture.

This mixture was then placed on a rectangular tray (25.5 x 19.5 x 4.5cm) and polymerised at 70°C during 18 hours under nitrogen atmosphere. The resulting polymer was then cut to the required shape and washed with RO water under gentle shaking to remove unreacted monomers, solvent and the NaCl. The sponge pieces were then placed in a solution containing 7% PEG 300 for 2 hours, drained and then dried at room temperature.

This method has also been performed with TFMAA (36g TFMAA used instead of the 48g of IA).

This method can also be performed using the monomers MBA, UA or MA, or any of the other monomers recited in Table 1.

Formulation with Zirconium:

Flexible cross-linked sponges containing Itaconic acid moieties and $ZrO_2$ were synthesised by first derivatising poly(vinyl butyral-co-vinyl acetate) (PBAA) with an average molecular weight of 50.00 - 80.00 with acryloyl chloride. For this purpose 24g PBAA was dissolved in 360ml of anhydrous methlpyrrolidone and 6ml acryloyl chloride add dropwise under stirring. The mixture was then allowed to react under stirring in the dark at 0°C for two hours.

After 2 hours 60g $ZrO_2$ (powder, 5$\mu$m particle size), 48g Itaconic acid, 60ml poly(ethylene glycol) diacrylate (average $M_n$ 575) and 12g free-radial initiator 1,1'-azobis(cyclohexanecarbonitrile) were added to the mixture. In order to create large pores in the final sponge, 2.5Kg NaCl (fine cooking salt grade) were thoroughly mixed with the above monomer/$ZrO_2$ mixture.

This mixture was then placed on a rectangular tray (25.5 x 19.5 x 4.5cm) and polymerised at 70°C during 18 hours under nitrogen atmosphere. The resulting polymer was the cut to the required shape and washed with RO water under gentle shaking to remove unreacted monomers, solvent and the NaCl. The sponge pieces were then placed in a solution containing 7% PEG 300 for 2 hours, drained and then dried at room temperature.

This method can also be performed using the monomers TFMAA, MBA, UA or MA, or any of the other monomers recited in Table 1.

**Example 3: Polymer synthesis and composition (IA gel formulation)**

Gels containing linear IA polymer:

Linear IA polymer was prepared by adding DMF containing 0.12g/ml IA and 6.6 mg/ml 1,1'-azobis(cyclohexanecarbonitrile). The mixture was bubbled with nitrogen to remove most of the dissolved oxygen from solution and headspace, sealed with screw cap and polymerised at 80°C for 18 hours.

Polymer was precipitated by adding the polymerisation mixture to approximately 7 volumes of RO water. HCl 1 M was added drop wise ($\leq$0.4% total volume) under slow stirring to increase the rate of flocculation. Polymer aggregates were then filtered out and washed with 10 volumes RO water and dried at room temperature.

The linear polymer was then dispersed in PEG 400 at a concentration of 0.23g polymer/ml PEG.

Gels containing cross-linked IA polymer:

IA cross-linked polymer particles were prepared the same way as the powder formulations (Example 1) but with a 1.4:1 molar ratio of EGDMA:IA.

Particles were collected in the 20-45$\mu$m size range and dispersed in PEG400 at a concentration of 0.23g polymer/ ml PEG.

**Example 4: Evaluation**

General Methods and Materials

Chemical agents

Chemical warfare agent simulant methyl salicylate was purchased from Sigma chemical company (Poole, UK) and was reported to be >99% pure. Radiolabelled methyl salicylate with no solvent was purchased from ARC (UK) Ltd. (Cardiff, UK). A working solution with a nominal activity of $0.2\mu$Ci $\mu$l$^{-1}$ was made upon delivery and stored at 4°C. Ethanol and isopropanol (both analytical grade) were purchased from the Sigma Chemical Company (Dorset, UK). Liquid scintillation counting fluid (Ultima Gold) and scintillation counting vials (5ml) were purchased from Perkin Elmer LAS (UK) Ltd (Buckinghamshire, UK).

Chemical warfare (CW) agents ('VX'; S-[2-(diisopropylamino)ethyl]-O-ethyl methylphosphonothioate, soman; 'GD'; O-Pinacolyl methylphosphonofluoridate and sulphur mustard; 'HD'; bis(2-chloroethyl)sulphide), and their ($^{14}$C-) radiolabelled analogues, were custom synthesised by TNO Defense, Security and Safety (Rijswijk, Netherlands). All were reported to be >97% purity. Each radiolabelled CW agent was mixed with 5g of corresponding undiluted agent to provide a stock solution with a nominal activity of ~ 1 mCi ml$^{-1}$ and was stored for up to four months at 4°C. Aliquots of each stock solution were diluted with unlabelled CW agent immediately prior to each experiment to provide a working solution with a nominal activity of ~0.5 $\mu$Ci $\mu$l$^{-1}$. The storage and use of CW agents was in full compliance with the Chemical Weapons Convention (1986).

Skin Preparation

Full thickness, close-clipped pig skin was obtained post mortem from the dorsal aspect of five female animals (*Sus scrofa,* large white strain, weight range 20-30kg) purchased from a reputable supplier. The skin from each animal was stored flat between sheets of aluminium foil at -20°C for up to twelve weeks before use. For each experiment, skin from one animal was removed from cold storage and thawed in a refrigerator at 5°C for ~ 24 hours. The skin was then dermatomed (Humeca model D42, Eurosurgical Ltd, Guildford, UK) to a nominal depth of 500$\mu$m and cut into squares (~3 x 3 cm).

Diffusion cell preparation

Skin diffusion cells were purchased from PermeGear (Chicago, IL., USA) and comprised an upper (donor) and lower (receptor) chamber with an area available for diffusion of 1.76 cm$^2$. A section of dermatomed skin was placed between the two chambers (epidermal surface facing the donor chamber) and the ensemble was securely clamped. The receptor chambers were filled with fluid (50% (v/v) aqueous ethanol; 14 $\pm$ 0.8 ml) so that the meniscus in the sampling arm was level with the surface of the skin sample. Each diffusion cell was placed in a Perspex™ holder above a magnetic stirrer which constantly mixed the receptor fluid via a (12 x 6 mm) Teflon™-coated iron bar placed within the receptor chamber. The receptor chambers were of the "jacketed" variety through which warm (36°C) water was pumped from a circulating water heater (Model GD120, Grant Instruments, Cambridge, UK) via a manifold to ensure a constant skin surface temperature of ~ 32°C (as confirmed by infrared thermography (FLIR Model P640 camera, Cambridge, UK). Up to 36 diffusion cells were used in each experiment, with six treatment groups each comprising n=6 diffusion cells. Once assembled, the diffusion cells were left *in situ* for an equilibration period of 24 hours.

Application of dose

Each experiment was started by the addition of 10 $\mu$l of $^{14}$C-radiolabelled CW agent simulant methyl salicylate (0.2 $\mu$Ci $\mu$l$^{-1}$) or $^{14}$C-radiolabelled Soman (GD), Sulphur Mustard (HD) or VX ((0.5 $\mu$Ci $\mu$l$^{-1}$) to the skin surface of each diffusion cell.

Where appropriate, decontamination was conducted at a pre-defined time point (depending on experiment) post-exposure, by the addition of a 200mg (powder) bolus of test decontaminant product to each contaminated skin surface.

Receptor chamber fluid (250$\mu$l) was withdrawn from each diffusion cell at regular intervals up to 24hrs post-exposure and was placed into vial containing 5ml of liquid scintillation counting (LSC) fluid. Each receptor chamber was replenished with an equivalent volume of fresh receptor fluid to maintain a constant volume in the receptor chamber.

Twenty four hours post-exposure, test decontaminant products were recovered from each skin surface and placed into glass vials containing 20ml LSC fluid. Receptor chamber fluid was removed and placed into 20ml glass vials. Each skin surface was then swabbed with a dry gauze pad which was subsequently placed in 20ml isopropanol (sigma chemical company, Dorset, UK). Finally, the skin from each diffusion cell was removed, placed into pre-weighed vials and skin weighed; 10ml of Soluene-350 was then added.

Analysis

The amount of radioactivity in each sample was quantified using a Perkin Elmer Tri-Carb liquid scintillation counter (Model 2810 TR), using an analysis time of 2 minutes per sample and a pre-set quench curve specific to the brand of LSC fluid used in this study. The amount of radioactivity in each sample was converted to amount of $^{14}$C-radiolabelled methyl salicylate by comparison to the corresponding standards (measured simultaneously). Quantification of the amount of radiolabelled methyl salicylate recovered in each receptor chamber sample enabled a calculation of the cumulative dermal absorption of methyl salicylate over 24 hours. These were averaged at each time point for each treatment group and plotted as total amount penetrated ($\mu$g cm$^{-2}$) against time for each experiment.

**Example 5: *In vitro* evaluation of powder formulations (IA, TFMAA, MBA, UA and MA) against Methyl Salicylate (MeS)**

To assess efficacy of powder formulations, skin diffusion experiments were set up as outlined in the general methods (above). The experiment was commenced by the addition of 10$\mu$l $^{14}$C-radiolabelled Methyl Salicylate (0.2 $\mu$Ci $\mu$l$^{-1}$). Powder formulations of IA, TFMAA, MBA, UA and MA decontaminant were administered (200mg) to contaminated porcine skin five minutes post exposure.

Over the course of 24hrs, receptor chamber fluid (250$\mu$l) was withdrawn from each diffusion cell at regular intervals and was placed into vials containing 5ml of liquid scintillation counting (LSC) fluid. Each receptor chamber was replenished with an equivalent volume of fresh receptor fluid to maintain a constant volume in the receptor chamber. To assess product efficacy, the powder formulations were left *in situ* for the duration of the experiment (24hrs) to ensure that the powders fully bound the contaminant and that the contaminant did not leach from the product.

Figure 2 illustrates the cumulative amount of $^{14}$C-radiolabelled agent MeS that penetrated the decontaminated pig skin over a 24hr period (as compared to untreated control pig skin). All points are mean $\pm$ standard deviation of n=6 diffusion cells.

**Example 6: *In vitro* evaluation of powder formulations (IA, TFMAA and MBA) against Soman (GD), Sulphur Mustard (HD) and VX.**

The most efficacious powder formulations from Example 5 (IA, TFMAA and MBA) were taken forward to be evaluated against Fullers' Earth (current UK military countermeasure) and a proprietary product (Fast Act).

To assess efficacy of powder formulations, skin diffusion experiments were set up as outlined in the general methods. The experiment commenced by the addition of 10$\mu$l $^{14}$C-radiolabelled HD, GD or VX (0.5 $\mu$Ci $\mu$l$^{-1}$).Powder formulations of IA, TFMAA, MBA, Fast Act and Fullers' Earth were administered (200mg) to contaminated porcine skin five minutes post exposure. Over the course of 24hours, receptor chamber fluid (250$\mu$l) was withdrawn from each diffusion cell at regular intervals and was placed into vials containing 5ml of liquid scintillation counting (LSC) fluid. Each receptor chamber was replenished with an equivalent volume of fresh receptor fluid to maintain a constant volume in the receptor chamber. To assess product efficacy the powder formulations were left *in situ* for the duration of the experiment (24hrs) to ensure the powders fully bind the contaminant and does not leach from the product. All points are mean $\pm$ standard deviation of n=6 diffusion cells.

Figures 3-5 illustrate the cumulative amount of $^{14}$C-radiolabelled agent Sulphur Mustard (Fig. 3), Soman (Fig. 4) or VX (Fig. 5) that penetrated the decontaminated pig skin over a 24hr period (as compared to untreated control pig skin).

**Example 7: Evaluation of IA and TFMAA Sponge formulations**

The most efficacious powder formulations from Example 6 (IA and TFMAA) were taken forward to be formulated into sponges.

To evaluate the efficacy of the sponge formulations (without Zirconium), the skin diffusion experiments were set up as outlined in the general methods. TFMAA and IA were formulated into sponges (as outlined in Example 2) and assessed against $^{14}$C-radiolabelled methyl salicylate.

The experiment commenced by the addition of 10$\mu$l $^{14}$C-radiolabelled methyl salicylate (0.2 $\mu$Ci $\mu$l$^{-1}$). Sponge formulations of TFMAA or IA were administered to contaminated porcine skin at 5, 30, 60, 180 and 360 minutes post exposure. Sponge discs having a diameter of 15mm, 0.5mm thickness and average weight of 150mg$\pm$30mg were placed on the contaminated skin surface for no longer than 5 seconds each side (total 10 seconds), then the sponge discs were removed and placed into 20ml glass vials. Over the course of 24hrs, receptor chamber fluid (250$\mu$l) was withdrawn from

each diffusion cell at regular intervals and was placed into vials containing 5ml of liquid scintillation counting (LSC) fluid. Each receptor chamber was replenished with an equivalent volume of fresh receptor fluid to maintain a constant volume in the receptor chamber.

To assess the effects of delayed decontamination and the ability of the product to prevent further absorption of the contaminant through the skin, the sponge was applied as described above (10 second contact time) and removed.

All points are mean ± standard deviation of n=6 diffusion cells.

Figures 6-7 illustrate the cumulative amount of $^{14}$C-radiolabelled MeS that penetrated the decontaminated pig skin over a 24 hour period (as compared to untreated control pig skin) - TFMAA (Fig 6); IA (Fig 7).

Figure 8 illustrates the cumulative amount of $^{14}$C-radiolabelled Sulphur Mustard that penetrated the IA-sponge decontaminated pig skin over a 24 hour period (as compared to untreated control pig skin).

### Example 7: Efficacy of IA sponge (with Zirconium) against GD, VX & HD

IA sponges with Zirconium were manufactured as outlined in Example 2.

To evaluate the efficacy of the sponge formulations with Zirconium, the skin diffusion experiments were set up as outlined in the general methods. The experiment commenced by the addition of 10μl $^{14}$C-radiolabelled Sulphur Mustard, Soman or VX (0.5 μCi μl[-1]). IA-Zr sponges were applied to contaminated porcine skin at 5 minutes post exposure. Sponge discs of a diameter of 15mm, 0.5mm thickness and average weight of 150mg±30mg were placed on the contaminated skin surface for no longer than 5 seconds each side (total 10 seconds) and then the sponge disc removed and placed into a 20ml glass vials. Over the course of 24hrs, receptor chamber fluid (250μl) was withdrawn from each diffusion cell at regular intervals and was placed into vials containing 5ml of liquid scintillation counting (LSC) fluid. Each receptor chamber was replenished with an equivalent volume of fresh receptor fluid to maintain a constant volume in the receptor chamber.

The IA-Zr sponges were applied as described above (10 second contact time) and removed to allow for direct comparison with the IA sponge experiments (Table 2).

All points are mean ± standard deviation of n=6 diffusion cells.

Figures 9-11 illustrate the cumulative amount of $^{14}$C-radiolabelled Sulphur Mustard (HD), Soman (GD) or VX (Figs 9-11 respectively) penetrating decontaminated (HD-SP) pig skin over a 24 hour period (as compared to untreated control pig skin).

$$\%CD24 = \frac{\text{Amount penetrated (Treated skin) at 24 hours}}{\text{Amount penetrated (Control skin) at 24 hours}} \times 100$$

Table 2: Summary of efficacy of IA sponge vs. IA-Zr sponge expressed as a percentage of control dose at 24 hours (%CD$_{24}$). This is indicative of the efficacy of the sponge against the chemical warfare agents Sulphur Mustard (HD), Soman (GD) and VX. The results in this table are obtained from skin decontaminated at 5mins post exposure to allow the comparison between each formulation.

| | %CD$_{24}$ | |
|---|---|---|
| | **IA Sponge** | **IA-Zr Sponge** |
| **HD** | 2.7 | 4.6 |
| **GD** | 73.9 | 35.6 |
| **VX** | 40.5 | 24.2 |

These results show that the addition of Zr to the IA sponge formulation gives an improvement in decontamination of the organophosphorus compounds GD and VX with little compromise of the efficacy of HD decontamination.

### Example 8: Efficacy of IA Gel formulation

IA Gel formulation (linear) was manufactured as outlined in Example 3.

To assess efficacy of IA gel formulation, skin diffusion experiments were set up as outlined in the general methods. The experiment commenced by the addition of 10μl $^{14}$C-radiolabelled HD, GD or VX (0.5 μCi μl[-1]). Gel

formulation of IA was administered (200μl) to contaminated porcine skin five minutes post exposure. Over the course of 24hrs, receptor chamber fluid (250μl) was withdrawn from each diffusion cell at regular intervals and was placed into vials containing 5ml of liquid scintillation counting (LSC) fluid. Each receptor chamber was replenished with an equivalent volume of fresh receptor fluid to maintain a constant volume in the receptor chamber. To assess efficacy the gel formulation was left *in situ* for the duration of the experiment (24hrs) to ensure the gel fully binds the contaminant and does not enhance penetration. All points are mean ± standard deviation of n=6 diffusion cells.

Skin surface decontamination was conducted five minutes post-exposure using Gel formulations of IA.

Figures 12-14 illustrate the cumulative amount of $^{14}$C-radiolabelled Soman (GD), VX, or Sulphur Mustard (HD) (Figs 12-14 respectively) that penetrated decontaminated pig skin over a 24 hour period (as compared to untreated control pig skin).

## Claims

1. A decontaminant product for decontaminating a surface or object that is contaminated, or suspected to be contaminated, with a hazardous chemical agent, wherein the decontaminant product comprises polymeric material comprising a polymer of:

   (i) itaconic acid, or a derivative thereof such as an itaconic ester or an itaconic acid isomer;
   (ii) an acrylate monomer;
   (iii) urocanic acid, or a derivative thereof such as an ester thereof;
   (iv) a vinyl monomer; or
   (v) an amine monomer,

   wherein the decontaminant product is a sponge, gel, liquid, paste, ointment, lotion, cream, foam, or fabric product suitable for personal use.

2. A decontaminant product according to Claim 1, wherein the decontaminant product is in the form of shampoo, shower gel, hand-wash, body-wash or detergent.

3. A decontaminant product according to Claim 1 or 2, wherein the polymeric material further comprises zirconium.

4. A decontaminant product according to any preceding claim, wherein the polymeric material comprises a polymer of:

   (i) itaconic acid
   (ii) an acrylate monomer selected from the group consisting of 2-trifluoromethyl acrylic acid (TFMAA), methacrylic acid (MA), *N,N*-methylene bisacrylamide (MBA) 2-hydroxyethyl methacrylate, *N,N*-diethylamino ethyl methacrylate (DEAEM), acrylamido-2-methyl-1-propanesulfonic acid (AMPSA), ethylene glycol dimethacrylate (EGDMA), acrylic acid, acrylamide, acrylonitrile and acrolein, or a derivate thereof;
   (iii) urocanic acid, or a derivate thereof such as an ester thereof, such as urocanic acid ethyl ester;
   (iv) a vinyl monomer selected from the group consisting of 1-Vinylimidazole, $_p$-divinylbenzene, $_m$-divinylbenzene, 2-Vinylpyridine and 4-Vinylpyridine; or
   (v) allylamine.

5. A decontaminant product according to any preceding claim, wherein the polymeric material comprises a polymer of itaconic acid, 2-trifluoromethyl acrylic acid, methacrylic acid, *N,N*-methylene bisacrylamide or urocanic acid.

6. A decontaminant product according to any previous claim, wherein the polymer is a co-polymer, such as an itaconic acid co-polymer.

7. A decontamination product according to any preceding claim, wherein the polymeric material comprises a cross-linked polymer.

8. A decontamination kit comprising:

   (a) a decontaminant product according to any one of Claims 1-7; and
   (b) a sealable container for storage and/or disposal of the decontaminant product following use.

9. Use of a decontaminant product according to any of Claims 1-7 for decontaminating a surface or object that is contaminated, or suspected to be contaminated, with a hazardous chemical agent; wherein the surface or object is any part of the human or animal body, such as skin, hair or teeth; clothing; or personal items or equipment.

10. A method of decontaminating a surface or object that is contaminated, or suspected to be contaminated, with a hazardous chemical agent; comprising contacting the surface or object with a decontaminant product according to any of Claims 1-7; wherein the surface or object is any part of the human or animal body, such as skin, hair or teeth; clothing; or personal items or equipment.

11. Use accordingly to Claim 9, or a method according to Claim 10, wherein the hazardous chemical agent is a chemical warfare agent, or a chemical surrogate thereof, such as methyl salicylate.

12. The use or method according to Claim 11, wherein the chemical warfare agent is sulphur mustard, ethyl ({2-[bis(propan-2-yl)amino]ethyl}sulfanyl)(methyl)phosphinate) or soman.

13. Use according to Claim 9, or a method according to Claim 10, wherein the surface or object is human skin or hair.

14. A barrier product for reducing or protecting against contamination of a surface or object by a hazardous chemical agent;
wherein the barrier product comprises a protective cover, a protective garment, or a barrier cream, lotion, gel, foam or paste; and comprises polymeric material comprising a polymer of:

(i) itaconic acid; or a derivative thereof such as an itaconic ester or an itaconic acid isomer;
(ii) an acrylate monomer selected from the group consisting of 2-trifluoromethyl acrylic acid (TFMAA), methacrylic acid (MA), *N,N*-methylene bisacrylamide (MBA) 2-hydroxyethyl methacrylate, *N,N*-diethylamino ethyl methacrylate (DEAEM), acrylamido-2-methyl-1-propanesulfonic acid (AMPSA), ethylene glycol dimethacrylate(EGDMA), acrylic acid, acrylamide, acrylonitrile and acrolein, or a derivative thereof;
(iii) urocanic acid, or a derivate thereof such as an ester thereof, such as urocanic acid ethyl ester;
(iv) a vinyl monomer selected from the group consisting of 1-Vinylimidazole, $_p$-divinylbenzene, $_m$-divinylbenzene, 2-Vinylpyridine and 4-Vinylpyridine; or
(v) allylamine

; and wherein the surface or object is any part of the human or animal body, such as skin, hair or teeth; clothing; or personal items or equipment.

15. A method of protecting a surface or object from contamination by a hazardous agent, comprising applying a barrier product according to Claim 14 to the surface or object; wherein the surface or object is any part of the human or animal body, such as skin, hair or teeth; clothing; or personal items or equipment.

16. A barrier kit, comprising a barrier product according to Claim 14 and one or more of:

(i) a sealable and impermeable container for disposal of the barrier product following use;
(ii) a decontaminant product;
(iii) a towel, flannel, hairbrush, comb, toothbrush, soap, shampoo, detergent, protective clothing and/ or ventilator equipment; and/ or
(iv) a set of instructions for use.

**Patentansprüche**

1. Dekontaminationsprodukt zum Dekontaminieren einer Oberfläche oder eines Objekts, die/das mit einem gefährlichen chemischen Mittel kontaminiert oder vermutlich kontaminiert ist, wobei das Dekontaminationsprodukt polymeres Material umfasst, das ein Polymer enthält von:

i) Itaconsäure oder einem Derivat davon, wie zum Beispiel einem Itaconsäureester oder einem Itaconsäure-Isomer;
ii) einem Acrylatmonomer;
iii) Urocaninsäure oder einem Derivat davon, wie zum Beispiel einem Ester davon;

iv) einem Vinylmonomer; oder

v) einem Aminmonomer,

wobei das Dekontaminationsprodukt ein Schwamm, ein Gel, eine Flüssigkeit, eine Paste, eine Salbe, eine Lotion, eine Creme, ein Schaum oder ein Textilprodukt, geeignet zur persönlichen Verwendung ist.

2. Dekontaminationsprodukt nach Anspruch 1, wobei das Dekontaminationsprodukt in der Form von einem Schampon, Duschgel, Händewaschmittel, Körperwaschmittel oder Reinigungsmittel vorliegt.

3. Dekontaminationsprodukt nach Anspruch 1 oder 2, wobei das polymere Material weiter Zirconium umfasst.

4. Dekontaminationsprodukt nach einem der vorangehenden Ansprüche, wobei das polymere Material ein Polymer enthält von:

   i) Itaconsäure

   ii) einem Acrylatmonomer, ausgewählt aus der Gruppe, bestehend aus 2-Trifluormethylacrylsäure (TFMAA), Methacrylsäure (MA), *N,N*-Methylenbisacrylamid(MBA)-2-hydroxyethylmethacrylat, *N,N*-Diethylaminoethylmethacrylat (DEAEM), Acrylamido-2-methyl-1-propansulfonsäure (AMPSA), Ethylenglykoldimethacrylat (EGDMA), Acrylsäure, Acrylamid, Acrylnitril und Acrolein oder einem Derivat davon;

   iii) Urocaninsäure oder einem Derivat davon, wie zum Beispiel einem Ester davon, wie zum Beispiel Urocaninsäureethylester;

   iv) einem Vinylmonomer, ausgewählt aus der Gruppe, bestehend aus 1-Vinylimidazol, p-Divinylbenzol, m-Divinylbenzol, 2-Vinylpyridin und 4-Vinylpyridin; oder

   v) Allylamin.

5. Dekontaminationsprodukt nach einem der vorangehenden Ansprüche, wobei das polymere Material ein Polymer von Itaconsäure, 2-Trifluormethylacrylsäure, Methacrylsäure, *N,N*-Methylenbisacrylamid oder Urocaninsäure enthält.

6. Dekontaminationsprodukt nach einem der vorangehenden Ansprüche, wobei das Polymer ein Copolymer, wie zum Beispiel ein Itaconsäure-Copolymer, ist.

7. Dekontaminationsprodukt nach einem der vorangehenden Ansprüche, wobei das polymere Material ein vernetztes Polymer enthält.

8. Dekontaminationskit, umfassend:

   a) ein Dekontaminationsprodukt nach einem der Ansprüche 1 bis 7; und

   b) einen fest verschließbaren Behälter zur Lagerung und/oder Entsorgung des Dekontaminationsprodukts nach der Verwendung.

9. Verwendung eines Dekontaminationsprodukts nach einem der Ansprüche 1 bis 7 zum Dekontaminieren einer Oberfläche oder eines Objekts, die/das mit einem gefährlichen chemischen Mittel kontaminiert oder vermutlich kontaminiert ist; wobei die Oberfläche oder das Objekt irgendein Teil des menschlichen oder tierischen Körpers, wie zum Beispiel Haut, Haar oder Zähne; Kleidung; oder persönliche Gegenstände oder Ausrüstungen ist.

10. Verfahren zum Dekontaminieren einer Oberfläche oder eines Objekts, die/das mit einem gefährlichen chemischen Mittel kontaminiert oder vermutlich kontaminiert ist; umfassend das Inkontaktbringen der Oberfläche oder des Objekts mit einem Dekontaminationsprodukt nach einem der Ansprüche 1 bis 7; wobei die Oberfläche oder das Objekt irgendein Teil des menschlichen oder tierischen Körpers, wie zum Beispiel Haut, Haar oder Zähne; Kleidung; oder persönliche Gegenstände oder Ausrüstungen ist.

11. Verwendung nach Anspruch 9 oder ein Verfahren nach Anspruch 10, wobei das gefährliche chemische Mittel ein chemischer Kampfstoff oder ein chemischer Ersatzstoff davon, wie zum Beispiel Methylsalicylat, ist.

12. Verwendung oder Verfahren nach Anspruch 11, wobei der chemische Kampfstoff Schwefellost, Ethyl-({2-[bis(propan-2-yl)amino]ethyl}sulfanyl)(methyl)-phosphinat) oder Soman ist.

**13.** Verwendung nach Anspruch 9 oder ein Verfahren nach Anspruch 10, wobei die Oberfläche oder das Objekt Haut oder Haar vom Menschen ist.

**14.** Barriereprodukt zur Reduktion der oder zum Schutz gegen die Kontamination einer Oberfläche oder eines Objekts durch ein gefährliches chemisches Mittel; wobei das Barriereprodukt eine schützende Abdeckung, Schutzkleidung oder eine Barriere-Creme, eine -Lotion, ein -Gel, ein -Schaum oder eine -Paste umfasst; und polymeres Material umfasst, das ein Polymer enthält von:

i) Itaconsäure oder einem Derivat davon, wie zum Beispiel einem Itaconsäureester oder einem Itaconsäure-Isomer;
ii) einem Acrylatmonomer, ausgewählt aus der Gruppe, bestehend aus 2-Trifluormethylacrylsäure (TFMAA), Methacrylsäure (MA), *N,N*-Methylenbisacrylamid(MBA)-2-hydroxyethylmethacrylat, *N,N*-Diethylaminoethylmethacrylat (DEAEM), Acrylamido-2-methyl-1-propansulfonsäure (AMPSA), Ethylenglykoldimethacrylat (EGDMA), Acrylsäure, Acrylamid, Acrylnitril und Acrolein oder einem Derivat davon;
iii) Urocaninsäure oder einem Derivat davon, wie zum Beispiel einem Ester davon, wie zum Beispiel Urocaninsäureethylester;
iv) einem Vinylmonomer, ausgewählt aus der Gruppe, bestehend aus 1-Vinylimidazol, p-Divinylbenzol, m-Divinylbenzol, 2-Vinylpyridin und 4-Vinylpyridin; oder
v) Allylamin; und

wobei die Oberfläche oder das Objekt irgendein Teil des menschlichen oder tierischen Körpers, wie zum Beispiel Haut, Haar oder Zähne; Kleidung; oder persönliche Gegenstände oder Ausrüstungen ist.

**15.** Verfahren zum Schutz einer Oberfläche oder eines Objekts vor einer Kontamination durch ein gefährliches Mittel, umfassend die Applikation eines Barriereprodukts nach Anspruch 14 auf die Oberfläche oder das Objekt, wobei die Oberfläche oder das Objekt irgendein Teil des menschlichen oder tierischen Körpers, wie zum Beispiel Haut, Haar oder Zähne; Kleidung oder persönliche Gegenstände oder Ausrüstungen ist.

**16.** Barrierekit, umfassend ein Barriereprodukt nach Anspruch 14 und eines oder mehr von Folgendem:

i) einen fest verschließbaren und undurchlässigen Behälter zur Entsorgung des Barriereprodukts nach dem Gebrauch;
ii) ein Dekontaminationsprodukt;
iii) ein Handtuch, einen Waschlappen, eine Haarbürste, einen Kamm, eine Zahnbürste, Seife, Schampon, ein Reinigungsmittel, Schutzkleidung und/oder eine Ventilator-Ausrüstung; und/oder
iv) eine Gebrauchsanleitung.

**Revendications**

**1.** Produit décontaminant pour décontaminer une surface ou un objet qui est contaminé(e), ou suspecté(e) d'être contaminé(e), par un agent chimique dangereux, le produit décontaminant comprenant un matériau polymère comprenant un polymère :

(i) d'acide itaconique, ou d'un dérivé de celui-ci, tel qu'un ester itaconique ou un isomère d'acide itaconique ;
(ii) d'un monomère acrylate ;
(iii) d'acide urocanique, ou d'un dérivé de celui-ci, tel qu'un ester de celui-ci ;
(iv) d'un monomère vinylique ; ou
(v) d'un monomère amine,

le produit décontaminant étant une éponge, un gel, un liquide, une pâte, une pommade, une lotion, une crème, une mousse, ou un produit en tissu convenant pour un usage personnel.

**2.** Produit décontaminant selon la revendication 1, le produit décontaminant étant sous la forme d'un shampooing, d'un gel douche, d'un produit pour le lavage des mains, d'un produit pour le lavage du corps ou d'un détergent.

**3.** Produit décontaminant selon la revendication 1 ou 2, dans lequel le matériau polymère comprend en outre du zirconium.

**4.** Produit décontaminant selon l'une quelconque des revendications précédentes, dans lequel le matériau polymère comprend un polymère :

(i) d'acide itaconique ;
(ii) d'un monomère acrylate sélectionné dans le groupe constitué de l'acide 2-trifluorométhylacrylique (TFMAA), de l'acide méthacrylique (MA), du N,N-méthylènebisacrylamide (MBA), du méthacrylate de 2-hydroxyéthyle, du méthacrylate de N,N-diéthylaminoéthyle (DEAEM), de l'acide acrylamido-2-méthyl-1-propanesulfonique (AMPSA), du diméthacrylate d'éthylène glycol (EGDMA), de l'acide acrylique, de l'acrylamide, de l'acrylonitrile et de l'acroléine, ou d'un dérivé de ceux-ci ;
(iii) d'acide urocanique, ou d'un dérivé de celui-ci, tel qu'un ester de celui-ci, tel que l'ester éthylique d'acide urocanique ;
(iv) d'un monomère vinylique sélectionné dans le groupe constitué du 1-vinylimidazole, du p-divinylbenzène, du m-divinylbenzène, de la 2-vinylpyridine et de la 4-vinylpyridine ; ou
(v) d'allylamine.

**5.** Produit décontaminant selon l'une quelconque des revendications précédentes, dans lequel le matériau polymère comprend un polymère d'acide itaconique, d'acide 2-trifluorométhylacrylique, d'acide méthacrylique, de N,N-méthylènebisacrylamide ou d'acide urocanique.

**6.** Produit décontaminant selon l'une quelconque des revendications précédentes, dans lequel le polymère est un copolymère, tel qu'un copolymère d'acide itaconique.

**7.** Produit de décontamination selon l'une quelconque des revendications précédentes, dans lequel le matériau polymère comprend un polymère réticulé.

**8.** Kit de décontamination comprenant :

(a) un produit décontaminant selon l'une quelconque des revendications 1 à 7 ; et
(b) un récipient pouvant être fermé hermétiquement pour le stockage et/ou l'élimination du produit décontaminant après l'utilisation.

**9.** Utilisation d'un produit décontaminant selon l'une quelconque des revendications 1 à 7 pour décontaminer une surface ou un objet qui est contaminé(e), ou suspecté(e) d'être contaminé(e), par un agent chimique dangereux ; dans laquelle la surface ou l'objet est une partie quelconque du corps humain ou animal, telle que la peau, les cheveux ou les dents ; un vêtement ; ou des articles ou équipements personnels.

**10.** Procédé de décontamination d'une surface ou d'un objet qui est contaminé(e), ou suspecté(e) d'être contaminé(e), par un agent chimique dangereux, comprenant la mise en contact de la surface ou de l'objet avec un produit décontaminant selon l'une quelconque des revendications 1 à 7 ; dans lequel la surface ou l'objet est une partie quelconque du corps humain ou animal, telle que la peau, les cheveux ou les dents ; un vêtement ; ou des articles ou équipements personnels.

**11.** Utilisation selon la revendication 9, ou procédé selon la revendication 10, dans laquelle ou dans lequel l'agent chimique dangereux est un agent de guerre chimique, ou un substitut chimique de celui-ci, tel que le salicylate de méthyle.

**12.** Utilisation ou procédé selon la revendication 11, dans laquelle ou dans lequel l'agent de guerre chimique est le gaz moutarde, l'éthyl({2-[bis(propan-2-yl)amino]éthyl}sulfanyl)(méthyl)phosphinate) ou le soman.

**13.** Utilisation selon la revendication 9, ou procédé selon la revendication 10, dans laquelle ou dans lequel la surface ou l'objet est de la peau ou des cheveux humains.

**14.** Produit barrière pour réduire ou protéger contre la contamination d'une surface ou d'un objet par un agent chimique dangereux ;
le produit barrière comprenant un revêtement de protection, un vêtement de protection, ou une crème, une lotion, un gel, une mousse ou une pâte barrière ; et comprenant un matériau polymère comprenant un polymère :

(i) d'acide itaconique, ou d'un dérivé de celui-ci, tel qu'un ester itaconique ou un isomère d'acide itaconique ;

(ii) d'un monomère acrylate sélectionné dans le groupe constitué de l'acide 2-trifluorométhylacrylique (TFMAA), de l'acide méthacrylique (MA), du N,N-méthylènebisacrylamide (MBA), du méthacrylate de 2-hydroxyéthyle, du méthacrylate de N,N-diéthylaminoéthyle (DEAEM), de l'acide acrylamido-2-méthyl-1-propanesulfonique (AMPSA), du diméthacrylate d'éthylène glycol (EGDMA), de l'acide acrylique, de l'acrylamide, de l'acrylonitrile et de l'acroléine, ou d'un dérivé de ceux-ci ;

(iii) d'acide urocanique, ou d'un dérivé de celui-ci, tel qu'un ester de celui-ci, tel que l'ester éthylique d'acide urocanique ;

(iv) d'un monomère vinylique sélectionné dans le groupe constitué du 1-vinylimidazole, du p-divinylbenzène, du m-divinylbenzène, de la 2-vinylpyridine et de la 4-vinylpyridine ; ou

(v) d'allylamine ;

(vi) et dans lequel la surface ou l'objet est une partie quelconque du corps humain ou animal, telle que la peau, les cheveux ou les dents ; un vêtement ; ou des articles ou équipements personnels.

15. Procédé de protection d'une surface ou d'un objet contre une contamination par un agent dangereux, comprenant l'application d'un produit barrière selon la revendication 14 sur la surface ou l'objet ; dans lequel la surface ou l'objet est une partie quelconque du corps humain ou animal, telle que la peau, les cheveux ou les dents ; un vêtement ; ou des articles ou équipements personnels.

16. Kit de barrière, comprenant un produit barrière selon la revendication 14 et un ou plusieurs de :

(i) un récipient pouvant être fermé hermétiquement et imperméable pour l'élimination du produit barrière après l'utilisation ;

(ii) un produit décontaminant ;

(iii) une serviette, un gant, une brosse à cheveux, un peigne, une brosse à dents, un savon, un shampooing, un détergent, des vêtements de protection et/ou un équipement de ventilation ; et/ou

(iv) un mode d'emploi.

poly(vinyl butyral-co-viny alcohol-co-vinyl acetate)
derivatised with acryloyl chloride

poly(ethylene glycol diacrylate)

itaconic acid

Free radical polymerisation
Solvent, porogen

Cross-linked polymeric material

EP 2 834 278 B1

IA gel vs VX

Control VX    VX-Gel

IA Gel vs HD

**EP 2 834 278 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008125887 A

- US 5100477 A

**Non-patent literature cited in the description**

- **REDDITHOTA et al.** Environmental Science and Pollution Research - International. Springer Berlin/Heidelberg, 30 December 2011, vol. 19, 1841-1851

- *Chemical Weapons Convention,* 1986